# EUROPEAN PATENT SPECIFICATION

(11) **EP 2 669 278 B1**
(45) Date of publication and mention of the grant of the patent: **27.05.2015**
(21) Application number: 12739535.8
(22) Date of filing: 25.01.2012
(51) Int. Cl.: C07D 311/78, C07C 323/21, C07C 323/38, C09K 9/02, G02B 5/23, G02C 7/10

(54) **CHROMENE COMPOUND**
CHROMENVERBINDUNG
COMPOSÉ DE CHROMÈNE

(30) Priority: 27.01.2011 JP 2011014786
(43) Date of publication of application: 04.12.2013
(73) Proprietor: Tokuyama Corporation, Shunan-shi, Yamaguchi 745-0053 (JP)
(72) Inventor: TAKAHASHI Toshiaki, Shunan-shi, Yamaguchi 745-0053 (JP); TAKENAKA Junji, Shunan-shi, Yamaguchi 745-0053 (JP); MOMODA Junji, Shunan-shi, Yamaguchi 745-0053 (JP); TERANISHI Kazuhiro, Shunan-shi, Yamaguchi 745-0053 (JP); IZUMI Shinobu, Shunan-shi, Yamaguchi 745-0053 (JP)
(74) Representative: Beckmann, Claus
(86) International application number: PCT/JP2012/052194
(87) International publication number: WO 2012/102409

(56) References cited:
- EP-A1- 1 184 379
- WO-A1-00/35902
- WO-A1-01/19813
- WO-A1-2005/028465
- WO-A1-2007/140058
- WO-A1-2010/150905
- WO-A1-2011/016582
- US-A1- 2009 309 076

## Description

### TECHNICAL FIELD

The present invention relates to a novel chromene compound, and an intermediate and use thereof. More specifically, it relates to a novel chromene compound which is useful as a photochromic compound for photochromic spectacle lenses and an intermediate and use thereof.

### BACKGROUND ART

Photochromism is the reversible function of a certain compound that it changes its color swiftly upon exposure to light including ultraviolet light such as sunlight or light from a mercury lamp and returns to its original color when it is put in the dark by stopping its exposure to light. A compound having this property is called "photochromic compound" and used as a material for photochromic plastic lenses.

For the photochromic compound used for this purpose, the following properties are required: (I) the degree of coloration at a visible light range before ultraviolet light is applied (to be referred to as "initial coloration" hereinafter) should be low, (II) the degree of coloration upon exposure to ultraviolet light (to be referred to as "color optical density" hereinafter) should be high, (III) the speed from the time when the application of ultraviolet light is started to the time when the color optical density reaches saturation (to be referred to as "color development sensitivity" hereinafter) should be high; (IV) the speed from the stoppage of the application of ultraviolet light to the time when the compound returns to its original state (to be referred to as "fading speed" hereinafter) should be high, (V) the repeat durability of this reversible function should be high, and (VI) the solubility in a monomer composition which will become a host material after curing of the photochromic compound should be high so that its dispersibility in the host material in use becomes high.

As the photochromic compound which can satisfy these requirements, there are known chromene compounds having an indeno (2,1-f) naphtho(1,2-b)pyran structure as the basic skeleton (refer to a pamphlet of International Laid-Open WO99/15518 and a pamphlet of International Laid-Open WO2001/60811).

It is preferred that a photochromic plastic lens comprising the photochromic compound should develop a color of a neutral tint such as gray or brown. A color of a neutral tint is obtained by mixing together several different kinds of photochromic compounds which develop different colors. Stated more specifically, it is obtained by mixing together a yellow to red photochromic compound (yellow compound) having a maximum absorption at 430 to 530 nm and a purple to blue photochromic compound (blue compound) having a maximum absorption at 550 to 650 nm.

However, when color control is carried out by this method, various problems occur due to the difference in photochromic properties between the compounds which have been mixed together. For example, when the repeat durability of the yellow compound is lower than that of the blue compound and the photochromic plastic lens is used for a long time, there occurs a problem that the developed color gradually changes to a color of a strong blue tint.

Further, when the color development sensitivity and fading speed of the yellow compound are lower than those of the blue compound, there arises a problem that color during development has a strong blue tint and color during fading has a strong yellow tint.

It is considered that this problem can be solved by using a single compound which has two or more absorption maximums at the time of exposure and develops a color of a neutral tint (double peak compound). It is known that the yellow compound is generally inferior to the blue compound in durability. Therefore, double peak compounds having a high yellow color optical density (absorption peak having a maximum absorption wavelength at 430 to 530 nm) are desired. Out of these, a compound having higher yellow color optical density (absorption peak having a maximum absorption wavelength at 430 to 530 nm) than blue color optical density (absorption peak having a maximum absorption wavelength at 550 to 650 nm) is desired in consideration of color control (the ratio of the yellow color optical density to the blue color optical density in the double peak compound may be referred to as "double peak characteristic" hereinafter).

As the photochromic compound having two or more absorption maximums at the time of color development (double peak compound), there are known compounds represented by the following formulas (A) to (D). However, these compounds have room for the improvement of the following points. That is, a chromene compound represented by the following formula (A) (refer to a pamphlet of International Laid-Open WO01/19813) has low fading speed and low repeat durability though its double peak characteristic is high.

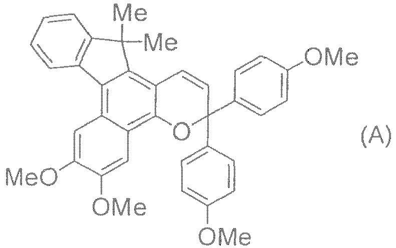

A chromene compound represented by the following formula (B) (refer to a pamphlet of International Laid-Open WO03/044022) has low double peak characteristic with a smaller absorption at 430 to 530 nm than an absorption at 550 to 650 nm.

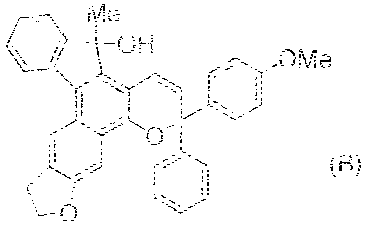

A chromene compound represented by the following formula (C) (refer to a pamphlet of International Laid-Open WO05/028465) has slightly strong initial coloration as the end of its absorption spectrum (to be referred to as "absorption end" hereinafter) before exposure goes beyond 420 nm into the visible range though it has excellent double peak characteristic and practical levels of color optical density and fading speed.

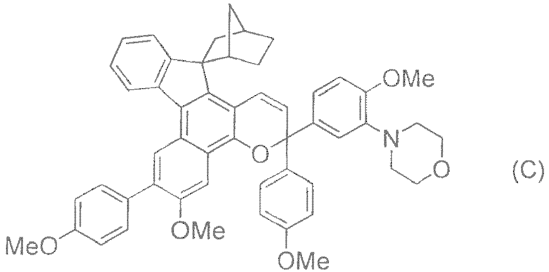

Further, a chromene compound represented by the following formula (D) which has a specific substituent at the 7-position is disclosed by US2009/0309076A1. However, this compound has low double peak characteristic with a smaller absorption at 430 to 530 nm than an absorption at 550 to 650 nm.

Additional chromene compounds with photochromic properties are disclosed in WO2010/150905, WO2007/140058, WO01/1983 and EP1184379.

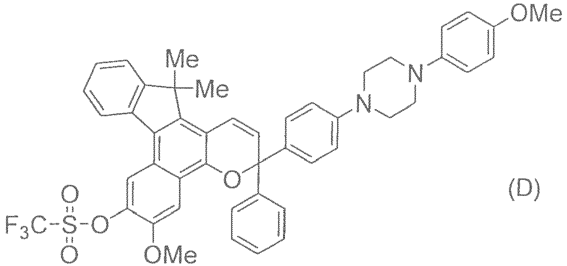

### DISCLOSURE OF THE INVENTION

It is therefore an object of the present invention to provide a chromene compound which develops a color of a neutral tint and has improved photochromic properties as compared with the above compounds.

It is another object of the present invention to provide a chromene compound which has little initial coloration, high color optical density and high fading speed, rarely colors at the time of deterioration and rarely experiences the reduction of color optical density when it is used repeatedly, that is, excellent durability of photochromic properties.

It is still another object of the present invention to provide a chromene compound which can be dissolved in a monomer composition which will become the substrate of an optical article in a high concentration.

It is a further object of the present invention to provide a novel naphthol compound for the manufacture of the chromene compound of the present invention.

It is known that a compound having a substituent having high electron donating ability (Hammett constant σₚ is smaller than 0) bonded to the 6-position and 7-position of an indeno(2,1-f)naphtho(1,2-b)pyran structure exhibits high double peak characteristic. The substituent having high electron donating ability as used herein is a substituent bonded to the 6-position and the 7-position via an oxygen atom or a nitrogen atom. Although the above compound has high double peak characteristic, it has defects such as low fading speed, great color development by heat at room temperature under no exposure (this color development will be referred to as "initial coloration by thermochromism" hereinafter) and low durability. Particularly when the electron donating abilities of the 6-position and 7-position substituents are further enhanced, the above defects become more marked. The Hammett constant σₚ is defined based on the Hammett equation that quantifies the electric effect of a substituent bonded to an n electron system on the basis of the dissociation constant Ka of p-substituted benzoic acid. A substituent having a σₚ of 0 is a hydrogen atom, and a substituent having a σₚ of less than 0 is a substituent having higher electron donating ability than a hydrogen atom.

Then, the inventors of the present invention conducted intensive studies to solve the above problems and found that a compound which has an aryl group or a heteroaryl group introduced into the 6-position and a specific substituent containing a sulfur atom introduced into the 7-position has excellent characteristic properties such as fading speed, initial coloration by thermochromism and durability while retaining high double peak characteristic though the electronic donating ability of the 7-positon substituent is low (Hammett constant σₚ is close to 0) and accomplish the present invention.

That is, firstly, the present invention is a chromene compound having a skeleton represented by the following formula (1): 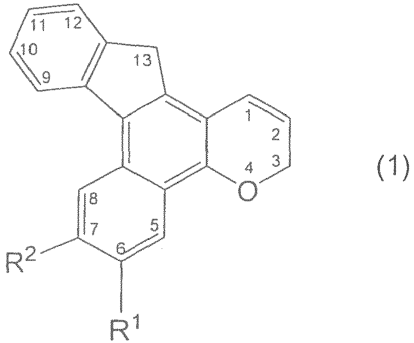 wherein R¹ is an aryl group or a heteroaryl group, and R² is a sulfur-containing substituent selected from the group consisting of thiol group, alkylthio group, alkoxyalkylthio group, haloalkylthio group, cycloalkylthio group, arylthio group and heteroarylthio group.

Secondly, the present invention is a photochromic curable composition comprising the above chromene compound of the present invention and polymerizable monomers.

Thirdly, the present invention is a photochromic optical article having a polymer molded product containing the chromene compound of the present invention dispersed therein as a constituent member. In the fourth place, the present invention is an optical article having an optical substrate all or part of at least one surface of which is covered with a polymer film containing the chromene compound of the present invention dispersed therein as a constituent member.

Finally, the present invention is a naphthol compound represented by the formula (5) which will be given hereinafter.

### BEST MODE FOR CARRYING OUT THE INVENTION

The chromene compound of the present invention has an indeno(2,1-f)naphtho(1,2-b)pyran structure represented by the above formula (1) as the basic skeleton. It is known that the chromene compound having an indeno(2,1-f)naphtho(1,2-b)pyran structure as the basic skeleton exhibits excellent photochromic properties. The chromene compound having the above basic skeleton of the present invention has an aryl group or a heteroaryl group at the 6-position and a sulfur-containing substituent selected from the group consisting of thiol group, alkylthio group, alkoxyalkylthio group, haloalkylthio group, cycloalkylthio group, arylthio group and heteroarylthio group at the 7-position (the above substituent introduced into the 7-position of the pyran structure may be simply referred to as "sulfur-containing substituent" hereinafter), thereby making it possible to develop a dark color of a neutral tint by itself while retaining excellent photochromic properties. A description is first given of R¹ and R² in the formula (1).

### <R¹>

R¹ which is a substituent bonded to the 6-position of the indeno(2,1-f)naphtho(1,2-b)pyran structure is an aryl group or a heteroaryl group.

The aryl group is preferably an aryl group having 6 to 14 carbon atoms. Preferred examples of the aryl group include phenyl group, 1-naphthyl group, 2-naphthyl group, 1-phenanthryl group, 2-phenanthryl group, 1-anthryl group and 9-anthryl group.

The heteroaryl group is preferably a heteroaryl group having 4 to 12 carbon atoms. The heteroaryl group is a group which forms a carbon-carbon bond together with the 6-position carbon atom. Preferred examples of the heteroaryl group include thienyl group, furyl group, pyrrolinyl group, pyridyl group, benzothienyl group, benzofuryl group and benzopyrrolinyl group.

The hydrogen atoms, preferably 1 to 4 hydrogen atoms of the above aryl group or the above heteroaryl group may be substituted by a hydroxyl group, alkyl group, haloalkyl group, cycloalkyl group, alkoxy group, amino group, heterocyclic group having a ring member nitrogen atom and bonded to an aryl group or a heteroaryl group bonded thereto via the nitrogen atom, cyano group, nitro group, formyl group, hydroxycarbonyl group, alkylcarbonyl group, alkoxycarbonyl group, halogen atom, aralkyl group, aralkoxy group, aryl group or aryloxy group. A description is subsequently given of these substituents.

### <substituent of R¹>

The alkyl group is preferably an alkyl group having 1 to 6 carbon atoms. Preferred examples of the alkyl group include methyl group, ethyl group, n-propyl group, isopropyl group, n-butyl group, sec-butyl group, tert-butyl group, pentyl group and hexyl group.

The haloalkyl group is preferably an alkyl group having 1 to 6 carbon atoms and substituted by a fluorine atom, chlorine atom or bromine atom. Preferred examples of the haloalkyl group include trifluoromethyl group, pentafluoroethyl group, chloromethyl group, 2-chloroethyl group and bromomethyl group.

The cycloalkyl group is preferably a cycloalkyl group having 3 to 8 carbon atoms. Preferred examples of the cycloalkyl group include cyclopropyl group, cyclobutyl group, cyclopentyl group and cyclohexyl group.

The alkoxy group is preferably an alkoxy group having 1 to 6 carbon atoms. Preferred examples of the alkoxy group include methoxy group, ethoxy group, n-propoxy group, isopropoxy group, n-butoxy group, sec-butoxy group and tert-butoxy group.

The amino group is not limited to amino group (-NH₂), and one or two hydrogen atoms thereof may be substituted. Examples of the substituent of the amino group include alkyl group having 1 to 6 carbon atoms, alkoxy group having 1 to 6 carbon atoms, cycloalkyl group having 3 to 8 carbon atoms, aryl group having 6 to 10 carbon atoms and heteroaryl group having 4 to 12 carbon atoms. Examples of these substituents are the same as those enumerated for R¹ and its substituents. Preferred examples of the amino group include amino group, methylamino group, dimethylamino group, ethylamino group, diethylamino group, phenylamino group and diphenylamino group.

Preferred examples of the heterocyclic group having a ring member nitrogen atom and bonded to an aryl group or a heteroaryl group bonded thereto via the nitrogen atom include morpholino group, piperidino group, pyrrolidinyl group, piperazino group, N-methylpiperazino group and indolinyl group. Further, the heterocyclic group may have a group having a Hammett constant σₚ of less than 0 as a substituent. Examples of the substituent include alkyl groups such as methyl group. Preferred examples of the heterocyclic group having a substituent include 2, 6-dimethylmorpholino group, 2, 6-dimethylpiperidino group and 2,2,6,6-tetramethylpiperidino group.

The alkylcarbonyl group is preferably an alkylcarbonyl group having 2 to 7 carbon atoms. Preferred examples thereof include acetyl group and ethylcarbonyl group.

The alkoxycarbonyl group is preferably an alkoxycarbonyl group having 2 to 7 carbon atoms. Preferred examples thereof include methoxycarbonyl group and ethoxycarbonyl group.

Examples of the halogen atom are fluorine atom, chlorine atom, bromine atom and iodine atom.

The aralkyl group is preferably an aralkyl group having 7 to 11 carbon atoms. Preferred examples of the aralkyl group include benzyl group, phenylethyl group, phenylpropyl group, phenylbutyl group and naphthylmethyl group.

The aralkoxy group is preferably an aralkoxy group having 7 to 11 carbon atoms. Preferred examples of the aralkoxy group include benzyloxy group and naphthylmethoxy group.

The aryl group is preferably an aryl group having 6 to 10 carbon atoms. Preferred examples of the aryl group include phenyl group, 1-naphthyl group and 2-naphthyl group.

The aryloxy group is preferably an aryloxy group having 6 to 10 carbon atoms. Preferred examples of the aryloxy group include phenoxy group, 1-naphthoxy group and 2-naphthoxy group.

### <preferred R¹>

Out of the groups R¹, an aryl group having 6 to 14 carbon atoms is preferred because it provides a compound having excellent double peak characteristic. To obtain especially excellent double peak characteristic and high color optical density, 1 to 4 hydrogen atoms of the aryl group are substituted preferably by a substituent having a Hammett constant σₚ of -1.00 or more to less than 0, particularly preferably by a substituent having a Hammett constant σₚ of -1.00 or more to less than -0.2. Specific examples of the aryl group include aryl groups substituted by at least one substituent selected from the group consisting of hydroxyl group, alkoxy group, amino group, heterocyclic group having a ring member nitrogen atom and bonded to an aryl group bonded thereto via the nitrogen atom, aralkoxy group and aryloxy group. Out of these, an aryl group substituted by at least one substituent selected from the group consisting of hydroxyl group, alkoxy group, amino group, heterocyclic group having a ring member nitrogen atom and bonded to an aryl group bonded thereto via the nitrogen atom and aryloxy group is preferred. An aryl group substituted by at least one substituent selected from the group consisting of alkoxy group, amino group and heterocyclic group having a ring member nitrogen atom and bonded to an aryl group bonded thereto via the nitrogen atom is more preferred. Particularly preferred examples of the aryl group include 4-methoxyphenyl group, 2,4-dimethoxyphenyl group, 4-(N,N-dimethylamino)phenyl group and 4-morphpolinophenyl group.

### <R²>

R² is a sulfur-containing substituent selected from the group consisting of thiol group, alkylthio group, alkoxyalkylthio group, haloalkylthio group, cycloalkylthio group, arylthio group and heteroarylthio group.

R² representing any one of these sulfur-containing substituents may also be represented by the formula R⁰²-S-(R⁰² is a hydrogen atom, alkyl group, alkoxyalkyl group, haloalkyl group, cycloalkyl group, aryl group or heteroaryl group).

The above alkylthio group is preferably an alkylthio group having 1 to 6 carbon atoms. Preferred examples of the alkylthio group include methylthio group, ethylthio group, n-propylthio group, isopropylthio group, n-butylthio group, sec-butylthio group and tert-butylthio group.

The above alkoxyalkylthio group is preferably an alkylthio group having 1 to 6 carbon atoms and substituted by an alkoxy group having 1 to 3 carbon atoms. Preferred examples of the alkoxyalkylthio group include methoxymethylthio group, methoxyethylthio group, methoxyn-propylthio group, methoxyn-butylthio group, ethoxyethylthio group and n-propoxypropylthio group.

The above haloalkylthio group is preferably an alkyl group having 1 to 6 carbon atoms and substituted by a fluorine atom, chlorine atom or bromine atom. Preferred examples of the haloalkylthio group include trifluoromethylthio group, tetrafluoroethylthio group, chloromethylthio group, 2-chloroethylthio group and bromomethylthio group.

The above cycloalkylthio group is preferably a cycloalkylthio group having 3 to 8 carbon atoms. Preferred examples of the cycloalkylthio group include cyclopropylthio group, cyclobutylthio group, cyclopentylthio group and cyclohexylthio group.

The above arylthio group is preferably an arylthio group having 6 to 10 carbon atoms. Preferred examples of the arylthio group include phenylthio group, 1-naphthylthio group and 2-naphthylthio group.

The above heteroarylthio group is preferably a heteroarylthio group having 4 to 12 carbon atoms. Preferred examples of the heteroarylthio group include thienylthio group, furylthio group, pyrrolylthio group, pyridylthio group, benzothienylthio group, benzofurylthio group and benzopyrrolylthio group.

One to nine hydrogen atoms, particularly preferably one to four hydrogen atoms of each of the above arylthio group and the above heteroarylthio group may be substituted by an alkyl group having 1 to 6 carbon atoms, an alkoxy group having 1 to 6 carbon atoms, a cycloalkyl group having 3 to 8 carbon atoms, or a halogen atom.

Out of the above sulfur-containing substituents, an alkylthio group having 1 to 6 carbon atoms, an alkylthio group having 1 to 6 carbon atoms and substituted by an alkoxy group having 1 to 3 carbon atoms, or a cycloalkylthio group having 3 to 8 carbon atoms is preferred as it enhances color optical density and double peak characteristic and reduces initial coloration by positioning the absorption end at a short wavelength range and the suppression of thermochromism. Further, an alkylthio group having 1 to 6 carbon atoms or a cycloalkylthio group having 3 to 8 carbon atoms is particularly preferred as it enhances color optical density and double peak characteristic in particular. Particularly preferred examples of these groups include methylthio group, ethylthio group and cyclohexylthio group.

A thiol group or a haloalkylthio group having 1 to 6 carbon atoms is preferred as it increases the fading speed and reduces initial coloration by thermochromism. Further, a haloalkylthio group having 1 to 6 carbon atoms is particularly preferred. More specifically, a trifluoromethylthio group is particularly preferred.

### <preferred chromene compound>

Out of the chromene compounds of the present invention, a chromene compound represented by the following formula (2) is preferred as it develops a color of a neutral tint and has high color optical density, high fading speed and high durability of photochromic properties.

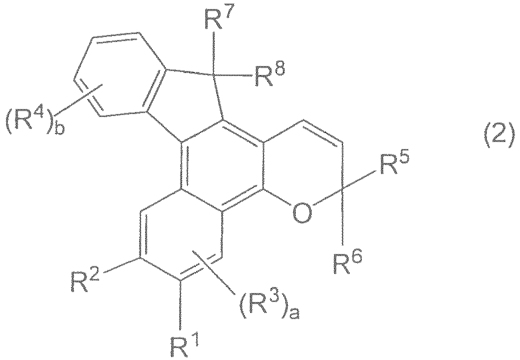

The substituents of the chromene compound represented by the above formula (2) will be described hereinbelow.

### <R¹>

In the above formula (2), R¹ is an aryl group or a heteroaryl group. These groups have already been explained. <R²>

In the above formula (2), R² is a sulfur-containing substituent. The substituent has already been explained.

### <R³ >

R³ is a hydroxyl group, alkyl group, haloalkyl group, cycloalkyl group, alkoxy group, amino group, heterocyclic group having a ring member nitrogen atom and bonded to a benzene ring bonded thereto via the nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group.

Out of these, R³ is preferably an alkyl group having 1 to 6 carbon atoms, haloalkyl group having 1 to 6 carbon atoms, cycloalkyl group having 3 to 15 carbon atoms, alkoxy group having 1 to 15 carbon atoms, amino group, heterocyclic group having a ring member nitrogen atom and bonded to a benzene ring bonded thereto via the nitrogen atom, alkylcarbonyl group having 2 to 7 carbon atoms, alkoxycarbonyl group having 2 to 7 carbon atoms, halogen atom, aralkyl group having 7 to 11 carbon atoms, aralkoxy group having 7 to 11 carbon atoms, aryloxy group having 6 to 10 carbon atoms or aryl group having 6 to 10 carbon atoms. Examples of R³ are the same as those enumerated for R¹ and the substituent of R¹.

R³ is particularly preferably a stereoscopically small group so as to provide high fading speed.

"a" is an integer of 0 to 2 indicative of the number of the group R³' s. When "a" is 2, two R³' s may be the same or different.

R³ preferably has a stereoscopically small substituent so as to obtain high fading speed. Particularly preferably, "a" is 0.

Even when there are a plurality of R³' s, preferred R³'s are same as those enumerated above.

### <R⁴>

R⁴ is a hydroxyl group, alkyl group, haloalkyl group, cycloalkyl group, alkoxy group, amino group, heterocyclic group having a ring member nitrogen atom and bonded to a benzene ring bonded thereto via the nitrogen atom, cyano group, nitro group, formyl group, hydroxycarbonyl group, alkylcarbonyl group, alkoxycarbonyl group, halogen atom, aralkyl group, aralkoxy group, aryl group, aryloxy group, thiol group, alkylthio group, alkoxyalkylthio group, haloalkylthio group, cycloalkylthio group, arylthio group, heteroarylthio group, hydroxysulfonyl group, alkylsulfonyl group, alkoxyalkylsulfonyl group, haloalkylsulfonyl group, cycloalkylsulfonyl group, arylsulfonyl group, heteroarylsulfonyl group, hydroxysulfinyl group, alkylsulfinyl group, alkoxyalkylsulfinyl group, haloalkylsulfinyl group, cycloalkylsulfinyl group, arylsulfinyl group and heteroarylsulfinyl group.

Examples of the above alkyl group, haloalkyl group, cycloalkyl group, alkoxy group, amino group, heterocyclic group having a ring member nitrogen atom and bonded to a benzene ring bonded thereto via the nitrogen atom, alkylcarbonyl group, alkoxycarbonyl group, halogen atom, aralkyl group, aralkoxy group, aryl group and aryloxy group are the same as those enumerated for the above R³. Preferred examples thereof are the same as those enumerated for the above R³.

The above alkylthio group is preferably an alkylthio group having 1 to 6 carbon atoms. Preferred examples of the alkylthio group include methylthio group, ethylthio group, n-propylthio group, isopropylthio group, n-butylthio group, sec-butylthio group and t-butylthio group.

The above alkoxyalkylthio group is preferably an alkylthio group having 1 to 6 carbon atoms and substituted by an alkoxy group having 1 to 3 carbon atoms. Preferred examples of the alkoxyalkylthio group include methoxymethylthio group, methoxyethylthio group, methoxyn-propylthio group, methoxyn-butylthio group, ethoxyethylthio group and n-propoxypropylthio group.

The above haloalkylthio group is preferably an alkylthio group having 1 to 6 carbon atoms and substituted by a fluorine atom, chlorine atom or bromine atom. Preferred examples of the haloalkylthio group include trifluoromethylthio group, tetrafluoroethylthio group, chloromethylthio group, 2-chloroethylthio group and bromomethylthio group.

The above cycloalkylthio group is preferably a cycloalkylthio group having 3 to 8 carbon atoms. Preferred examples of the cycloalkylthio group include cyclopropylthio group, cyclobutylthio group, cyclopentylthio group and cyclohexylthio group.

The above arylthio group is preferably an arylthio group having 6 to 10 carbon atoms. Preferred examples of the arylthio group include phenylthio group, 1-naphthylthio group and 2-naphthylthio group.

The above heteroarylthio group is preferably a heteroarylthio group having 4 to 12 carbon atoms. Preferred examples of the heteroarylthio group include thienylthio group, furylthio group, pyrrolylthio group, pyridylthio group, benzothienylthio group, benzofurylthio group and benzopyrrolylthio group.

One to nine hydrogen atoms, particularly preferably one to four hydrogen atoms of each of the above arylthio group and the above heteroarylthio group may be substituted by an alkyl group having 1 to 6 carbon atoms, an alkoxy group having 1 to 6 carbon atoms, a cycloalkyl group having 3 to 8 carbon atoms, or a halogen atom.

The above alkylsulfonyl group is preferably an alkylsulfonyl group having 1 to 6 carbon atoms. Preferred examples of the alkylsulfonyl group include methylsulfonyl group, ethylsulfonyl group, n-propylsulfonyl group, isopropylsulfonyl group, n-butylsulfonyl group, sec-butylsulfonyl group and t-butylsulfonyl group.

The above alkoxyalkylsulfonyl group is preferably an alkylsulfonyl group having 1 to 6 carbon atoms and substituted by an alkoxy group having 1 to 3 carbon atoms. Preferred examples of the alkoxyalkylsulfonyl group include methoxymethylsulfonyl group, methoxyethylsulfonyl group, methoxyn-propylsulfonyl group, methoxyn-butylsulfonyl group, ethoxyethylsulfonyl group and n-propoxypropylsulfonyl group.

The above haloalkylsulfonyl group is preferably an alkylsulfonyl group having 1 to 6 carbon atoms and substituted by a fluorine atom, chlorine atom or bromine atom. Preferred examples of the haloalkylsulfonyl group include trifluoromethylsulfonyl group, tetrafluoroethylsulfonyl group, chloromethylsulfonyl group, 2-chloroethylsulfonyl group and bromomethylsulfonyl group.

The above cycloalkylsulfonyl group is preferably a cycloalkylsulfonyl group having 3 to 8 carbon atoms. Preferred examples of the cycloalkylsulfonyl group include cyclopropylsulfonyl group, cyclobutylsulfonyl group, cyclopentylsulfonyl group and cyclohexylsulfonyl group.

The above arylsulfonyl group is preferably an arylsulfonyl group having 6 to 10 carbon atoms. Preferred examples of the arylsulfonyl group include phenylsulfonyl group, 1-napthylsulfonyl group and 2-naphthylsulfonyl group.

The above heteroarylsulfonyl group is preferably a heteroarylsulfonyl group having 4 to 12 carbon atoms. Preferred examples of the heteroarylsulfonyl group include thienylsulfonyl group, furylsulfonyl group, pyrrolylsulfonyl group, pyridylsulfonyl group, benzothienylsulfonyl group, benzofurylsulfonyl group and benzopyrrolylsulfonyl group.

One to nine hydrogen atoms, particularly preferably one to four hydrogen atoms of each of the above arylsulfonyl group and the above heteroarylsulfonyl group may be substituted by an alkyl group having 1 to 6 carbon atoms, an alkoxy group having 1 to 6 carbon atoms, a cycloalkyl group having 3 to 8 carbon atoms, or a halogen atom.

The above alkylsulfinyl group is preferably an alkylsulfinyl group having 1 to 6 carbon atoms. Preferred examples of the alkylsulfinyl group include methylsulfinyl group, ethylsulfinyl group, n-propylsulfinyl group, isopropylsulfinyl group, n-butylsulfinyl group, sec-butylsulfinyl group and t-butylsulfinyl group.

The above alkoxyalkylsulfinyl group is preferably an alkylsulfinyl group having 1 to 6 carbon atoms and substituted by an alkoxy group having 1 to 3 carbon atoms. Preferred examples of the alkoxyalkylsulfinyl group include methoxymethylsulfinyl group, methoxyethylsulfinyl group, methoxyn-propylsulfinyl group, methoxyn-butylsulfinyl group, ethoxyethylsulfinyl group and n-propoxypropylsulfinyl group.

The above haloalkylsulfinyl group is preferably an alkylsulfinyl group having 1 to 6 carbon atoms and substituted by a fluorine atom, chlorine atom or bromine atom. Preferred examples of the haloalkylsulfinyl group include trifluoromethylsulfinyl group, tetrafluoroethylsulfiyl group, chloromethylsulfinyl group, 2-chloroethylsulfinyl group and bromomethylsulfinyl group.

The above cycloalkylsulfinyl group is preferably a cycloalkylsulfinyl group having 3 to 8 carbon atoms. Preferred examples of the cycloalkylsulfinyl group include cyclopropylsulfinyl group, cyclobutylsulfinyl group, cyclopentylsulfinyl group and cyclohexylsulfinyl group.

The above arylsulfinyl group is preferably an arylsulfinyl group having 6 to 10 carbon atoms. Preferred examples of the arylsulfinyl group include phenylsulfinyl group, 1-naphthylsulfinyl group and 2-naphthylsulfinyl group.

The above heteroarylsulfinyl group is preferably a heteroarylsulfinyl group having 4 to 12 carbon atoms. Preferred examples of the heteroarylsulfinyl group include thienylsulfinyl group, furylsulfinyl group, pyrrolylsulfinyl group, pyridylsulfinyl group, benzothienylsulfinyl group, benzofurylsulfinyl group and benzopyrrolylsulfinyl group.

One to nine hydrogen atoms, particularly preferably one to four hydrogen atoms of each of the above arylsulfinyl group and the above heteroarylsulfinyl group may be substituted by an alkyl group having 1 to 6 carbon atoms, an alkoxy group having 1 to 6 carbon atoms, a cycloalkyl group having 3 to 8 carbon atoms, or a halogen atom.

"b" is an integer of 0 to 4 which indicates the number of R⁴' s. When "b" is an integer of 2 to 4, a plurality of R⁴' s may be the same or different.

Out of these, R⁴ is preferably a haloalkyl group, cyano group, alkoxycarbonyl group, alkylthio group, haloalkylthio group, cycloalkylthio group, alkylsulfonyl group, cycloalkylsulfonyl group, alkylsulfinyl group or cycloalkylsulfinyl group, particularly preferably a haloalkyl group, cyano group, alkylthio group or haloalkylthio group as excellent double peak characteristic and high fading speed are obtained. More specifically, R⁴ is particularly preferably a hydrogen atom, trifluoromethyl group, cyano group, methylthio group or tifluoromethylthio group. Further, a case where "b" is 0 is also particularly preferred. R⁴ is preferably bonded to the 11-position carbon atom in order to obtain higher fading speed.

Even when there are a plurality of R⁴' s, preferred R⁴' s are the same as those enumerated above.

### <R⁵ and R⁶>

R⁵ and R⁶ are each independently a group represented by the following formula (3), group represented by the following formula (4), aryl group, heteroaryl group or alkyl group.

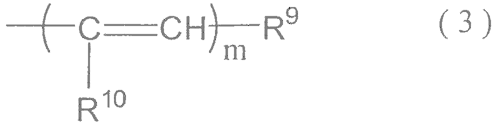

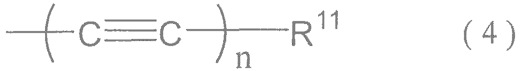

In the above formula (3), R⁹ is an aryl group or a heteroaryl group. Examples of the aryl group and the heteroaryl group are the same as those enumerated for R¹.

R¹⁰ is a hydrogen atom, alkyl group or halogen atom. Examples of the alkyl group include methyl group, ethyl group and propyl group. Examples of the halogen atom are fluorine atom, chlorine atom, bromine atom and iodine atom.

"m" is an integer of 1 to 3. From the viewpoint of the acquisition of the raw material, "m" is preferably 1.

Preferred examples of the group represented by the above formula (3) include phenyl-ethenyl group, (4-(N,N-dimethylamino)phenyl)-ethenyl group, (4-morpholinophenyl)-ethenyl group, (4-piperidinophenyl)-ethenyl group, (4-methoxyphenyl)-ethenyl group, (2-methoxyphenyl)-ethenyl group, phenyl-1-methylethenyl group, (4-methoxyphenyl)-1-methylethenyl group, phenyl-1-fluoroethenyl group, (4-(N,N,-dimethylamino)phenyl)-1-fluoroethenyl group, 2-thienyl-ethenyl group, 2-furyl-ethenyl group, 2-(N-methyl)pyrrolinyl-ethenyl group, 2-benzothienyl-ethenyl group, 2-benzofuranyl-ethenyl group and 2-(N-methyl)indolyl-ethenyl group.

In the above formula (4), R¹¹ is an aryl group or a heteroaryl group. Examples of these groups are the same as those enumerated for R⁹. "n" is an integer of 1 to 3. From the viewpoint of the easy acquisition of the raw material, "n" is preferably 1.

Preferred examples of the group represented by the above formula (4) include phenyl-ethynyl group, (4-(N,N-dimethylamino)phenyl)-ethynyl group, (4-morpholinophenyl)-ethynyl group, (4-piperidinophenyl)-ethynyl group, (4-methoxyphenyl)-ethynyl group, (4-methylphenyl)-ethynyl group, (2-methoxyphenyl)-ethynyl group, 2-thienyl-ethynyl group, 2-furyl-ethynyl group, 2-(N-methyl)pyrrolinyl-ethynyl group, 2-benzothienyl-ethyl group, 2-benzofuranyl-ethynyl group and 2-(N-methyl)indolyl-ethynyl group,

Examples of the aryl group and the heteroaryl group represented by R⁵ and R⁶ are the same as those enumerated for R¹. Examples of the alkyl group are the same as those enumerated as the substituent for R¹.

R⁵ and R⁶ may be bonded together to form an aliphatic hydrocarbon ring together with the carbon atom bonded thereto.

Preferred examples of the aliphatic hydrocarbon ring include adamantane ring, bicyclononane ring, norbornane ring and fluorene ring.

In order to obtain excellent photochromic properties (double peak characteristic and fading speed), desirably, at least one, preferably both of R⁵ and R⁶ are aryl groups or heteroaryl groups. Particularly preferably, at least one, preferably both of R⁵ and R⁶ are each any one of the groups (i) to (iii):
(i) an aryl group or heteroaryl group having an alkyl group or alkoxy group as a substituent;
(ii) an aryl group or heteroaryl group having an amino group as a substituent;
(iii) an aryl group or heteroaryl group having a heterocyclic group having a ring member nitrogen atom and bonded to an aryl group or heteroaryl group via the nitrogen atom as a substituent.

The substitution positions and the total number of substituents in the aryl groups (i) to (iii) are not particularly limited. In order to obtain excellent photochromic properties, when the aryl group is a phenyl group, the substitution position is preferably 3-position or 4-position, and the number of substituents is preferably 1 or 2. Examples of this aryl group include 4-methylphenyl group, 4-methoxyphenyl group, 3,4-dimethoxyphenyl group, 4-n-propoxyphenyl group, 4-(N,N-dimethylamino)phenyl group, 4-(N,N-diethylamino)phenyl group, 4-(N,N-diphenylamino)phenyl group, 4-morpholinophenyl group, 4-piperidinophenyl group, 3-(N,N-dimethylamino)phenyl group and 4-(2,6-dimethylpiperidino)phenyl group.

The substitution positions and the total number of substituents in the heteroaryl groups (i) to (iii) are not particularly limited. The number of the substituents is preferably 1. Preferred examples of the heteroaryl group include 4-methoxythienyl group, 4-(N,N-dimethylamino)thienyl group, 4-methylfuryl group, 4-(N,N-diethylamino)furyl group, 4-(N,N-diphenylamino)thienyl group, 4-morpholinopyrrolinyl group, 6-piperidinobenzothienyl group and 6-(N,N-dimethylamino)benzofuranyl group.

### <R⁷ and R⁸>

R⁷ and R⁸ are each independently a hydrogen atom, hydroxyl group, alkyl group, haloalkyl group, cycloalkyl group, alkoxy group, amino group, heterocyclic group having a ring member nitrogen atom and bonded to the 13-positon carbon atom via the nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group.

Out of the above groups, R⁷ and R⁸ are each preferably an alkyl group having 1 to 6 carbon atoms, haloalkyl group having 1 to 6 carbon atoms, cycloalkyl group having 3 to 15 carbon atoms, alkoxy group having 1 to 15 carbon atoms, amino group, heterocyclic group having a ring member nitrogen atom and bonded to the 13-positon carbon atom via the nitrogen atom, alkylcarbony group having 2 to 7 carbon atoms, alkoxycarbonyl group having 2 to 7 carbon atoms, halogen atom, aralkyl group having 7 to 11 carbon atoms, aralkoxy group having 7 to 11 carbon atoms, aryloxy group having 6 to 10 carbon atoms or aryl group having 6 to 10 carbon atoms.

Examples thereof are the same as those enumerated for R¹, the substituent of R¹, and R².

R⁷ and R⁸ may be bonded together to form an aliphatic ring having 3 to 20 ring member carbon atoms, condensed polycyclic ring having an aromatic ring or aromatic hetero ring condensed to the aliphatic ring, hetero ring having 3 to 20 ring member atoms, or condensed polycyclic ring having an aromatic ring or aromatic hetero ring condensed to the hetero ring, together with the 13-position carbon atom bonded thereto.

Examples of the above aliphatic ring having 3 to 20 ring member carbon atoms include cyclopentane ring, cyclohexane ring, cyclooctane ring, cycloheptane ring, norbornane ring, bicyclononane ring and adamantane ring.

Examples of the condensed polycyclic ring having an aromatic ring or aromatic hetero ring condensed to the above aliphatic ring include phenanthrene ring.

Examples of the above hetero ring having 3 to 20 ring member atoms include dihydrothiophene ring, dihydrofuran ring, tetrahydrofuran ring and dihydropyridine ring.

Examples of the above condensed polycyclic ring having an aromatic ring or aromatic hetero ring condensed to the above hetero ring include dihydrobenzofuran ring and dihydrobenzothiophene ring.

### <particularly preferred R⁷ and R⁸>

In the present invention, preferred substituents R⁷ and R⁸ are each a hydroxyl group, alkyl group, alkoxy group or substituent which forms a ring together with the 13-position carbon atom bonded thereto. An example of the alkyl group is methyl group and an example of the alkoxy group is methoxy group. To increase the fading speed and reduce initial coloration by thermochromism while retaining high color optical density and high double peak characteristic, out of the above preferred substituents, R⁷ and R⁸ are more preferably substituents which form a ring together with the 13-position carbon atom bonded thereto. They are particularly preferably substituents which form the above aliphatic ring or the condensed polycyclic ring having an aromatic ring or aromatic hetero ring condensed to the above aliphatic ring as the fading speed in particular becomes high. They are most preferably substituents which form the above aliphatic ring as it increases the fading speed most and reduces initial coloration by thermochromism.

The aliphatic ring formed by R⁷ and R⁸ is particularly preferably an aliphatic hydrocarbon ring having 3 to 20 ring member carbon atoms. This aliphatic hydrocarbon ring may have at least one substituent selected from the group consisting of alkyl group having 1 to 6 carbon atoms, haloalkyl group having 1 to 6 carbon atoms, cycloalkyl group having 3 to 15 carbon atoms, alkoxy group having 1 to 15 carbon atoms, amino group, aralkyl group having 7 to 11 carbon atoms, aryl group having 6 to 10 carbon atoms and halogen atom. The aliphatic hydrocarbon ring substituted like this is also preferred. Examples of the alkyl group, haloalkyl group, cycloalkyl group, alkoxy group, amino group, aralkyl group, aryl group and halogen atom are the same as those enumerated for R^{1,} the substituent of R¹, and R².

More preferred examples of the aliphatic hydrocarbon ring include monocyclic rings such as cyclopentane ring, cyclohexane ring, cycloheptane ring, cyclooctane ring and cyclononane ring, bicyclo rings such as norbornane ring and bicyclononane ring, and tricyclo rings such as adamantane ring. These rings having at least one lower alkyl group having 4 or less carbon atoms such as methyl group are also preferred. Out of these, monocyclic rings produce a particularly excellent effect as they reduce initial coloration by thermochromism while retaining high color optical density, high double peak characteristic and high fading speed.

In the present invention, most preferred typical examples of the ring formed by R⁷ and R⁸ bonded together with the 13-position carbon atom bonded thereto are represented by the following formulas. In the following formulas, the carbon atom denoted by 13 is the 13-position carbon atom of the above pyran structure.

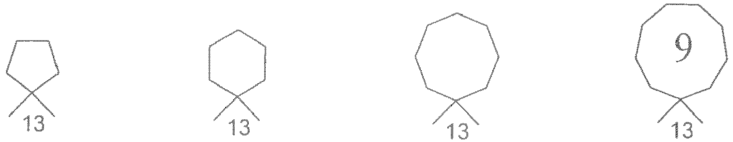

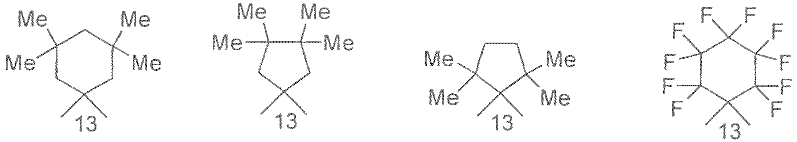

Out of the above monocyclic rings, a cyclooctane ring, cyclononane ring and 3,3,5,5-tetramethylcyclohexane ring are most preferred.

### <particularly preferred chromene compound>

Particularly preferred examples of the chromene compound in the present invention are the following compounds.

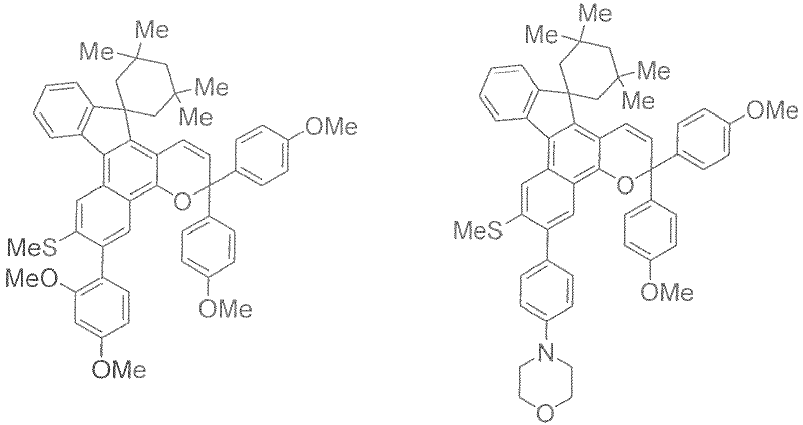

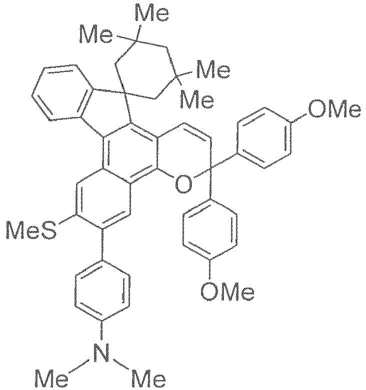

### (identification of chromene compound)

The chromene compound of the present invention is generally existent as an achromatic, light yellow or light green solid or viscous liquid at normal temperature and normal pressure and can be confirmed by the following means (1) to (3).
(1) When the proton nuclear magnetic resonance spectrum (¹H-NMR) of the chromene compound is measured, peaks based on an aromatic proton and an alkene proton appear at δ of around 5.0 to 9.0 ppm and peaks based on the protons of an alkyl group and an alkylene group appear at δ of around 1.0 to 4.0 ppm. By comparing these spectral intensities relatively, the number of the protons of bonds can be known.
(2) The composition of a corresponding product can be determined by elemental analysis.
(3) When the ¹³C-nuclear magnetic resonance spectrum (¹³C-NMR) of the chromene compound is measured, a peak based on the carbon of an aromatic hydrocarbon group appears at δ of around 110 to 160 ppm, peaks based on the carbons of an alkene and an alkyne appear at δ of around 80 to 140 ppm, and peaks based on the carbons of an alkyl group and an alkylene group appear at δ of around 20 to 80 ppm.

### <production of chromene compound>

The chromene compound of the present invention may be produced by any synthesis process. For example, the chromene compound represented by the above formula (2) can be advantageously produced by the following process. The symbols in the following formulas are as defined in the above formulas unless otherwise noted.

The chromene compound can be advantageously produced by reacting a naphthol compound represented by the following formula (5) with a propargyl alcohol compound represented by the following formula (6) in the presence of an acid catalyst. 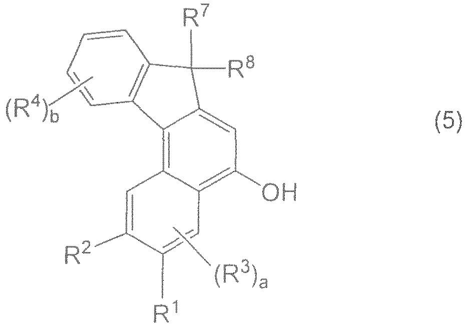 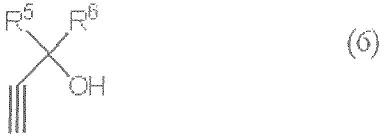 The reaction ratio of the naphthol compound to the propargyl alcohol compound is preferably selected from 1:10 to 10:1 (molar ratio). As the acid catalyst is used sulfuric acid, benzenesulfonic acid, p-toluenesulfonic acid or acid alumina. The acid catalyst is preferably used in an amount of 0.1 to 10 parts by weight based on 100 parts by weight of the total of the naphthol compound and the propargyl alcohol compound. The reaction temperature is preferably 0 to 200°C. An aprotic organic solvent such as N-methylpyrrolidone, dimethyl formamide, tetrahydrofuran, benzene or toluene is preferably used as the solvent. The method of purifying the product obtained through the above reaction is not particularly limited. For example, the obtained product may be purified by carrying out silica gel column purification and further recrystallization.

The naphthol compound represented by the above formula (5) is provided as a novel compound by the present invention.

Particularly preferred examples of the naphthol compound represented by the formula (5) are the following compounds.

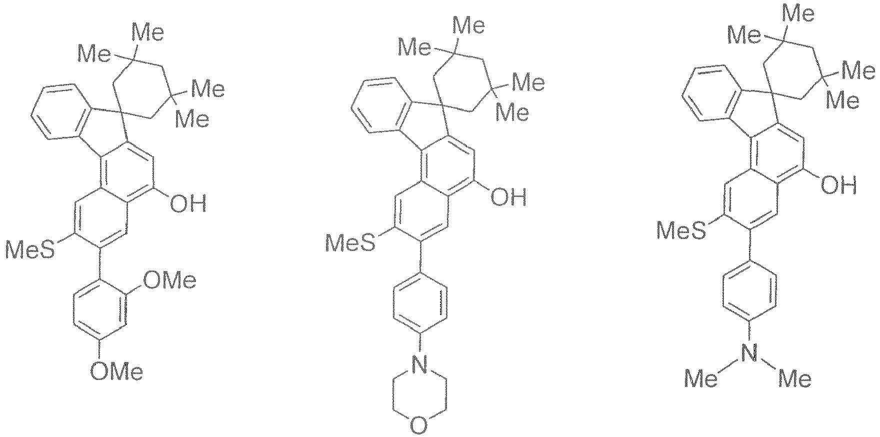

Ordinary naphthol compounds can be synthesized in accordance with reaction methods described in, for example, research papers such as a pamphlet of International Laid-Open WO01/60881 and a pamphlet of International Laid-Open WO05/028465.

The naphthol compound represented by the above formula (5) can be synthesized as follows, for example.

To begin with, a benzene compound represented by the following formula (7) may be purchased as a commercially available product or may be synthesized based on the following documents. 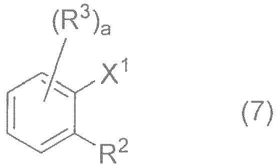 (R², R³ and "a" are as defined in the above formula (2). X¹ is a chlorine atom, bromine atom, iodine atom or the same as R¹ in the formula (2).)

For example, a benzene compound represented by the following formula (8) can be purchased as a commercially available product.

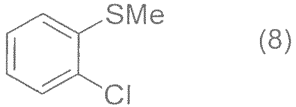

For example, a benzene compound represented by the following formula (9) can be synthesized in accordance with a reaction method described in research papers such as Journal of Materials Chemistry, 2095-2100; 1999.

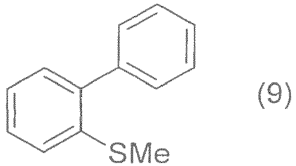

A compound represented by the following formula (10) (R⁴ and "b" are as defined in the above formula (2)) is produced by reacting the compound (7) with acid chloride and then when X¹ is a chlorine atom, bromine atom or iodine atom, it is converted into desired R¹ by using a Suzuki-Miura reaction to obtain a compound represented by the following formula (11).

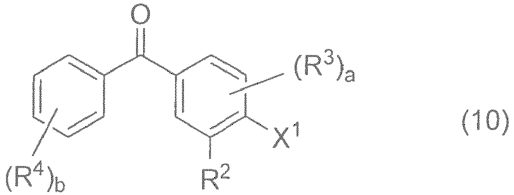

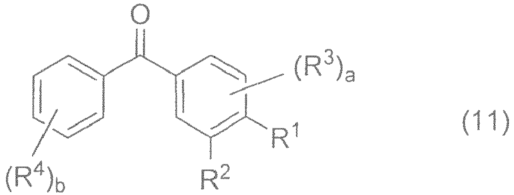

A compound represented by the following formula (12) is obtained by subjecting the above compound (11) to a Stobbe reaction and a cyclization reaction. 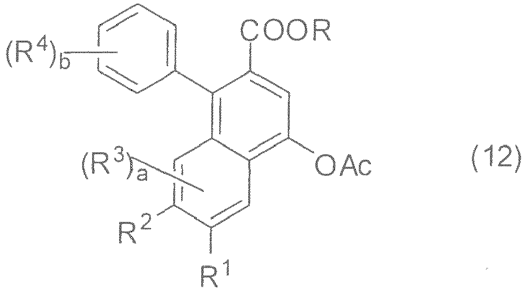 In the compound of the formula (12), R is a group derived from a diester compound used in the Stobbe reaction, and Ac is an acetyl group. The compound (12) is then hydrolyzed by using an alkali or acid to obtain a carboxylic acid represented by the following formula (13). 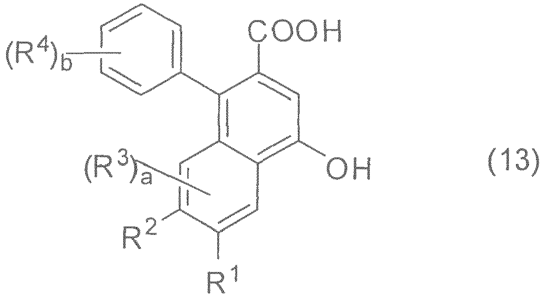 This carboxylic acid is benzylated by using a base such as potassium carbonate and benzyl chloride and then hydrolyzed by using an alkali or acid to obtain a benzyl-protected carboxylic acid represented by the following formula (14) (Bn in the formula (14) is a benzyl group). 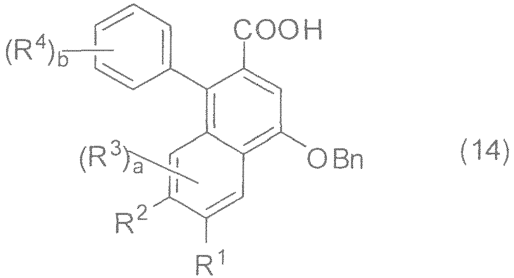 This benzyl-protected carboxylic acid is converted into an amine by a method such as Curtius rearrangement, Hofmann rearrangement or Lossen rearrangement, and a diazonium salt is prepared from the amine by a method known per se. This diazonium salt is converted into a bromide through a Sandmeyer reaction or the like, and the obtained bromide is reacted with magnesium or lithium to prepare an organic metal compound. This organic metal compound is reacted with a ketone represented by the following formula (15) at -80 to 70°C in an organic solvent for 10 minutes to 4 hours and then a debenzylation reaction is carried out by using hydrogen and palladium carbon to obtain an alcohol represented by the following formula (16). 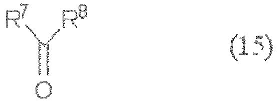 (R⁷ and R⁸ are as defined in the above formula (2).) 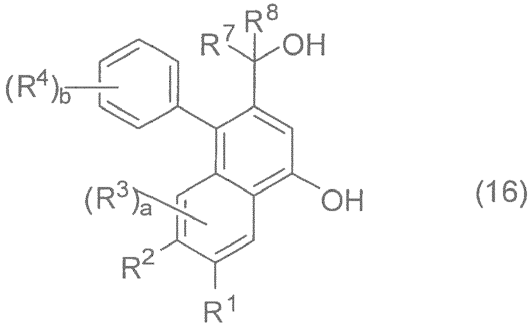 This alcohol is subjected to a Friedel-Crafts reaction at 10 to 120°C for 10 minutes to 2 hours under a neutral to acid condition so as to synthesize a naphthol compound of the above formula (5) of interest. In the above reaction, the reaction ratio of the above organic metal compound to the ketone represented by the above formula (15) is preferably selected from a range of 1:10 to 10:1 (molar ratio). The reaction temperature is preferably -80 to 70°C. An aprotic organic solvent such as diethyl ether, tetrahydrofuran, benzene or toluene is preferably used as the solvent. The Friedel-Crafts reaction of the alcohol of the above formula (16) under a neutral to acid condition is preferably carried out by using an acid catalyst such as acetic acid, hydrochloric acid, sulfuric acid, benzenesulfonic acid, p-toluenesulfonic acid or acid alumina. The acid catalyst is preferably used in an amount of 0.1 to 10 parts by weight based on 100 parts by weight of the alcohol of the above formula (16). For this reaction, an aprotic organic solvent such as tetrahydrofuran, benzene or toluene is used.

The propargyl alcohol compound represented by the above formula (6) can be easily synthesized by reacting a ketone compound corresponding to the above formula (6) with a metal acetylene compound such as lithium acetylide.

The chromene compound of the present invention which is synthesized as described above dissolves well in a general organic solvent such as toluene, chloroform or tetrahydrofuran. When the chromene compound represented by the above formula (1) is dissolved in this solvent, the obtained solution is generally almost achromatic and transparent and has an excellent photochromic function that it develops a color swiftly upon exposure to sunlight or ultraviolet radiation and reversibly returns to its original achromatic state swiftly by blocking off the light.

### <stabilizer>

Although the chromene compound of the present invention has high durability as it is, its durability can be further enhanced by using the following ultraviolet absorbent, optical stabilizer or antioxidant. As the ultraviolet absorbent may be used known ultraviolet absorbents such as benzophenone-based compounds, benzotriazole-based compounds, cyanoacrylate-based compounds, triazine-based compounds and benzoate-based compounds. Cyanoacrylate-based compounds and benzophenone-based compounds are particularly preferred. When the above ultraviolet stabilizer is used in an amount of 0.001 to 5 parts by mass based on 100 parts by mass of the total of all the polymerizable monomers including the chromene compound of the present invention, it produces an effect. Known hindered amines may be used as the optical stabilizer, and known hindered phenols may be used as the antioxidant. When the above optical stabilizer and antioxidant are each used in an amount of 0.01 to 10 parts by mass based on 100 parts by mass of the total of all the polymerizable monomers including the chromene compound of the present invention, they produce an effect.

### (use of chromene compound>

The chromene compound of the present invention exhibits the same photochromic properties even in a polymer solid matrix. The polymer solid matrix is not particularly limited if the chromene compound of the present invention can be uniformly dispersed therein. Examples of the optically preferred polymer compound for the polymer solid matrix include thermoplastic resins such as methyl polyacrylate, ethyl polyacrylate, methyl polymethacrylate, ethyl polymethacrylate, polystyrene, polyacrylonitrile, polyvinyl alcohol, polyacrylamide, poly(2-hydroxyethylmethacrylate), polydimethylsiloxane and polycarbonate.

The chromene compound of the present invention may be mixed with polymerizable monomers before polymerization to obtain a photochromic curable composition which is then polymerized and cured to obtain a photochromic composition. That is, a photochromic curable composition comprising the photochromic composition of the present invention and polymerizable monomers is polymerized and cured to obtain a cured material containing the photochromic composition uniformly dispersed therein.

A photochromic curable composition which is prepared by mixing together the photochromic composition comprising the chromene compound represented by the above formula (1) with the following polymerizable monomers (A1), (A2) and (A3) and provides a cured material having an L scale Rockwell hardness of not less than 60 is particularly preferred because it exhibits excellent photochromic properties such as high color optical density and high fading speed and obtains excellent substrate properties such as high hardness and high heat resistance:
(A1) a polymerizable monomer which has an L scale Rockwell hardness of a polymer obtained by homopolymerizing it of not more than 40,
(A2) a trifunctional or higher functional radically polymerizable monomer which has an L scale Rockwell hardness of a polymer obtained by homopolymerizing it of not less than 60, and
(A3) a bifunctional radically polymerizable monomer having an L scale Rockwell hardness of a polymer obtained by homopolymerizing it of not less than 60.
Examples of the component (A1) include acrylate compounds and methacrylate compounds such as glycidyl acrylate, glycidyl methacrylate, β-methylglycidyl methacrylate, bisphenol A-monoglycidyl ether-methacrylate, 4-glycidyloxy methacrylate, 3-(glycidyl-2-oxyethoxy)-2-hydroxypropyl methacrylate,
3-(glycidyloxy-1-isopropyloxy)-2-hydroxypropyl acrylate and 3-glycidyloxy-2-hydroxypropyloxy)-2-hydroxypropyl acrylate, and polyalkylene glycol compounds such as polyethylene glycol diacrylate.

Examples of the component (A2) include polyacrylate and polymethacrylate compounds such as trimethylolpropane trimethacrylate, urethane acrylates such as urethane oligomer tetramethacrylate, and polyester acrylates such as polyester oligomer hexaacrylate.

Examples of the component (A3) include polyacrylate and polymethacrylate compounds such as ethylene glycol diacrylate, diethylene glycol dimethacrylate, triethylene glycol dimethacrylate, tetraethylene glycol dimethacrylate, ethylene glycol bisglycidyl methacrylate, bisphenol A dimethacrylate, 2,2-bis(4-methacryloyloxyethoxyphenyl)propane and 2,2-bis(3,5-dibromo-4-methacryloyloxyethoxyphenyl) propane, all of which are polymerizable monomers described in a pamphlet of International Laid-Open WO2001/05854. The term "L scale Rockwell hardness" means hardness measured in accordance with JIS-B7726, and it can be easily judged whether the above hardness condition is satisfied or not by measuring the homopolymer of each monomer.

Copolymers obtained by copolymerizing the above polymerizable monomers with polymerizable monofunctional monomers may also be used as the above polymer matrix. The polymerizable monofunctional monomers include unsaturated carboxylic acids such as acrylic acid, methacrylic acid and maleic anhydride; polyallyl compounds such as diallyl phthalate, diallyl terephthalate, diallyl isophthalate, diallyl tartarate, diallyl epoxysuccinate, diallyl fumarate, diallyl chlorendate, diallyl hexaphthalate, diallyl carbonate, allyl diglycol carbonate and trimethylolpropane triallyl carbonate; polythioacrylate and polythiomethacrylate compounds such as 1,2-bis(methacryloylthio)ethane, bis(2-acryloylthioethyl)ether and 1,4-bis(methacryloylthiomethyl)benzene; acrylate and methacrylate compounds such as methyl acrylate, methyl methacrylate, benzyl methacrylate, phenyl methacrylate and 2-hydroxyethyl methacrylate; fumarate compounds such as diethyl fumarate and diphenyl fumarate; thioacrylate and thiomethacrylate compounds such as methyl thioacrylate, benzyl thioacrylate and benzyl thiomethacrylate; and vinyl compounds such as styrene, chlorostyrene, methyl styrene, vinyl naphthalene, α-methylstyrene dimer and bromostyrene. These monomers may be used alone or in combination of two or more, and the amount of each of the monomers may be suitably determined according to use purpose.

The method of dispersing the chromene compound of the present invention into the above polymer solid matrix is not particularly limited, and commonly used methods may be employed. The methods include one in which the above thermoplastic resin and the chromene compound are kneaded together while they are molten to disperse the chromene compound into the resin, one in which the chromene compound is dissolved in the above polymerizable monomers and a polymerization catalyst is added to polymerize the polymerizable monomers by heat or light so as to disperse the chromene compound into the resin, and one in which the surfaces of the above thermoplastic resin and the above thermosetting resin are dyed with the chromene compound to disperse the chromene compound into the resins.

### (other applications of chromene compound of the present invention)

The chromene compound of the present invention can be widely used in photochromic materials such as recording materials as substitutes for silver halide photosensitive materials, copy materials, printing photosensitive materials, recording materials for cathode ray tubes, photosensitive materials for lasers and photosensitive materials for holography. A photochromic material comprising the chromene compound of the present invention may also be used as a photochromic plastic lens material, optical filter material, display material or material for actinometers and ornaments.

For instance, when the chromene compound of the present invention is used in a photochromic lens, a method in which uniform light control performance is obtained, for example, a method in which a polymer film containing the photochromic material of the present invention uniformly dispersed therein is sandwiched between lenses, a method in which the chromene compound of the present invention is dispersed into the above polymerizable monomers and the polymerizable monomers are polymerized by a predetermined technique, or a method in which the chromene compound of the present invention is dissolved in, for example, silicone oil, the resulting solution is impregnated into the surface of a lens at 150 to 200°C over 10 to 60 minutes, and the surface is further coated with a curable substance to obtain a photochromic lens may be employed. Further, a method in which the above polymer film is formed on the surface of a lens and the surface is coated with a curable substance to obtain a photochromic lens may also be employed.

Moreover, a coating agent composed of a polymerization curable composition comprising the chromene compound of the present invention may be applied to the surface of a lens substrate and cured. At this point, the lens substrate may be subjected to a surface treatment with an alkaline solution or a plasma treatment in advance, and a primer may be further applied to improve adhesion between the substrate and the coating film (by carrying out or not carrying out the above surface treatment).

### Examples

The following examples are provided for the purpose of further illustrating the present invention but are in no way to be taken as limiting.

### Example 1

1.1 g (2.0 mmol) of the following naphthol compound (17) and 0.80 g (3.0 mmol) of the following propargyl alcohol compound (18) were dissolved in 60 ml of toluene, 0.02 g of p-toluenesulfonic acid was further added to the resulting solution, and the obtained mixture was stirred under reflux by heating for 1 hour. 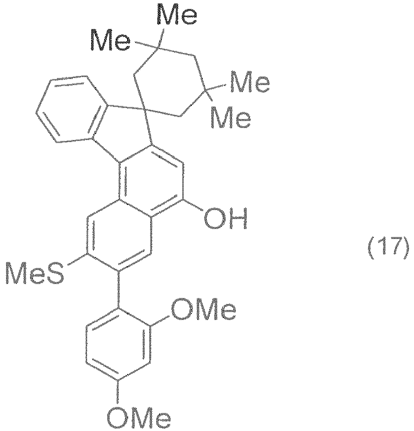 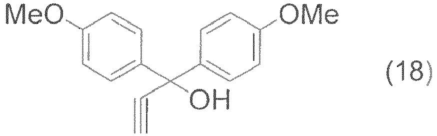 After a reaction, the solvent was removed, and the obtained product was purified on silica gel by chromatography to obtain 1.1 g of a white powder product. The yield was 72 %.

The elemental analysis values of this product were 79.26% of C, 6.80 % of H and 3.96 % of S which were almost equal to the calculated values of C₅₂H₅₂O₅S (C: 79.35 %, H: 6.64 %, S: 4.06 %)

When the proton nuclear magnetic resonance spectrum of the product was measured, it showed 18H peaks based on the methyl proton and methylene proton of a tetramethylcyclohexane ring at δ of around 1.0 to 3.0 ppm, 15H peaks based on the methyl proton of a methylthio group and the methyl proton of a methoxy group at δ of around 2.3 to 4.0 ppm and 19H peaks based on an aromatic proton and the proton of an alkene at δ of around 5.6 to 9.0 ppm.

Further, when the ¹³C-nuclear magnetic resonance spectrum was measured, it showed a peak based on the carbon of an aromatic ring at δ of around 110 to 160 ppm, a peak based on the carbon of an alkene at δ of around 80 to 140 ppm and a peak based on the carbon of an alkyl at δ of around 20 to 60 ppm.

It was confirmed from the above results that the isolated product was a compound represented by the following formula (19).

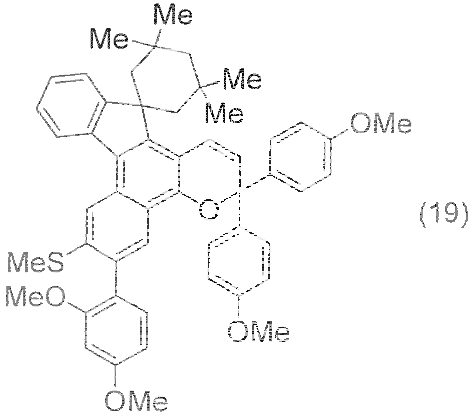

### Examples 2 to 28

Chromene compounds shown in Table 1 were synthesized in the same manner as in Example 1. When the structures of the obtained products were analyzed by using the same structure confirming means as in Example 1, it was confirmed that they were compounds represented by structural formulas shown in Tables 1 to 9. Tables 10 and 11 show the elemental analysis values, calculated values obtained from the structural formulas of the compounds and characteristic ¹H-NMR spectra of these compounds.

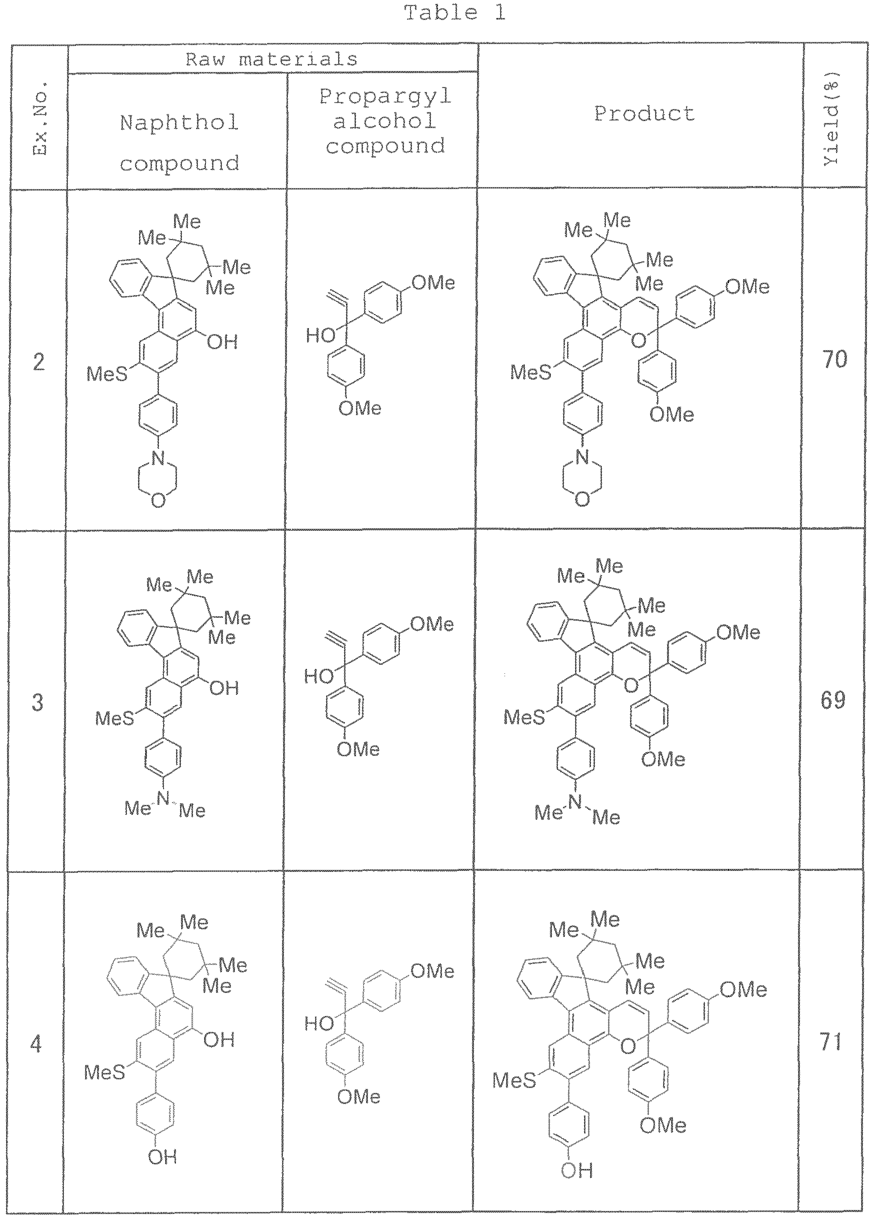

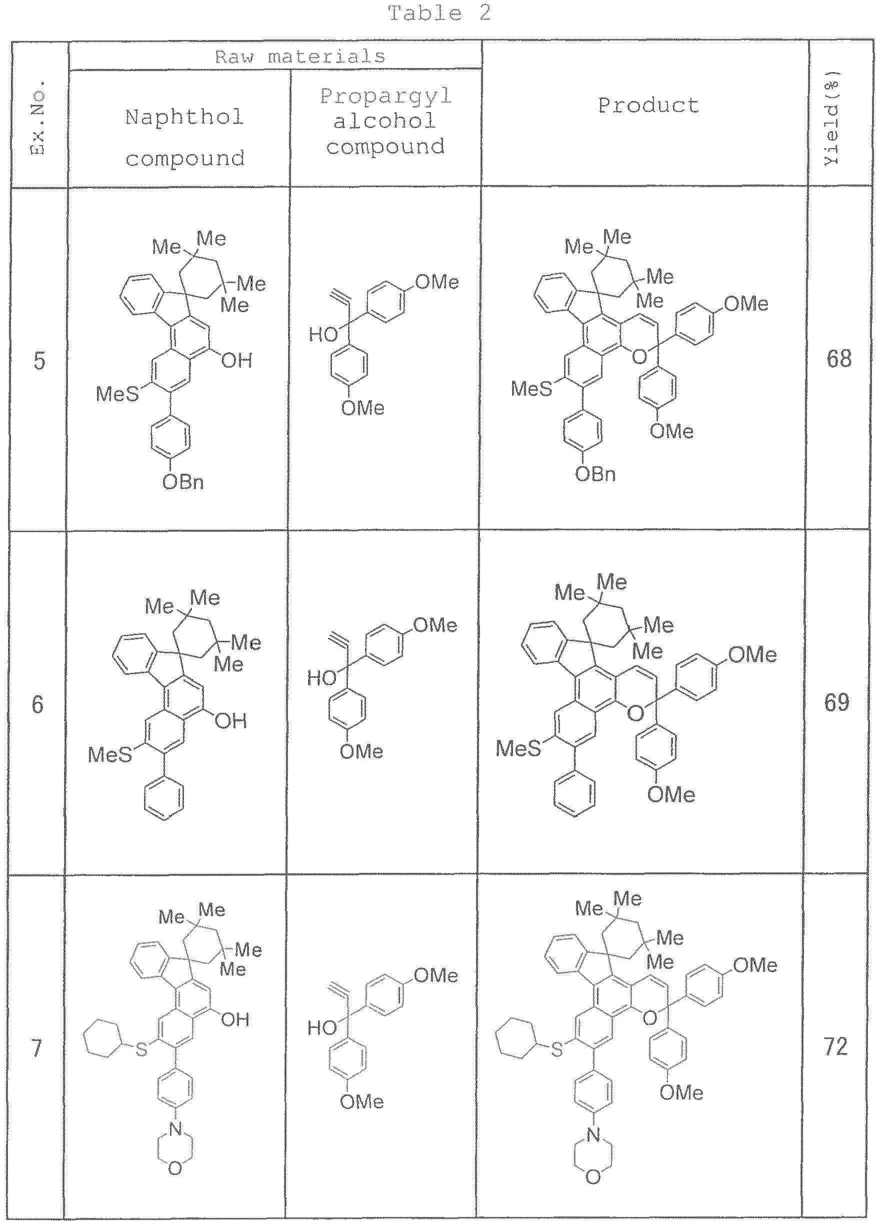

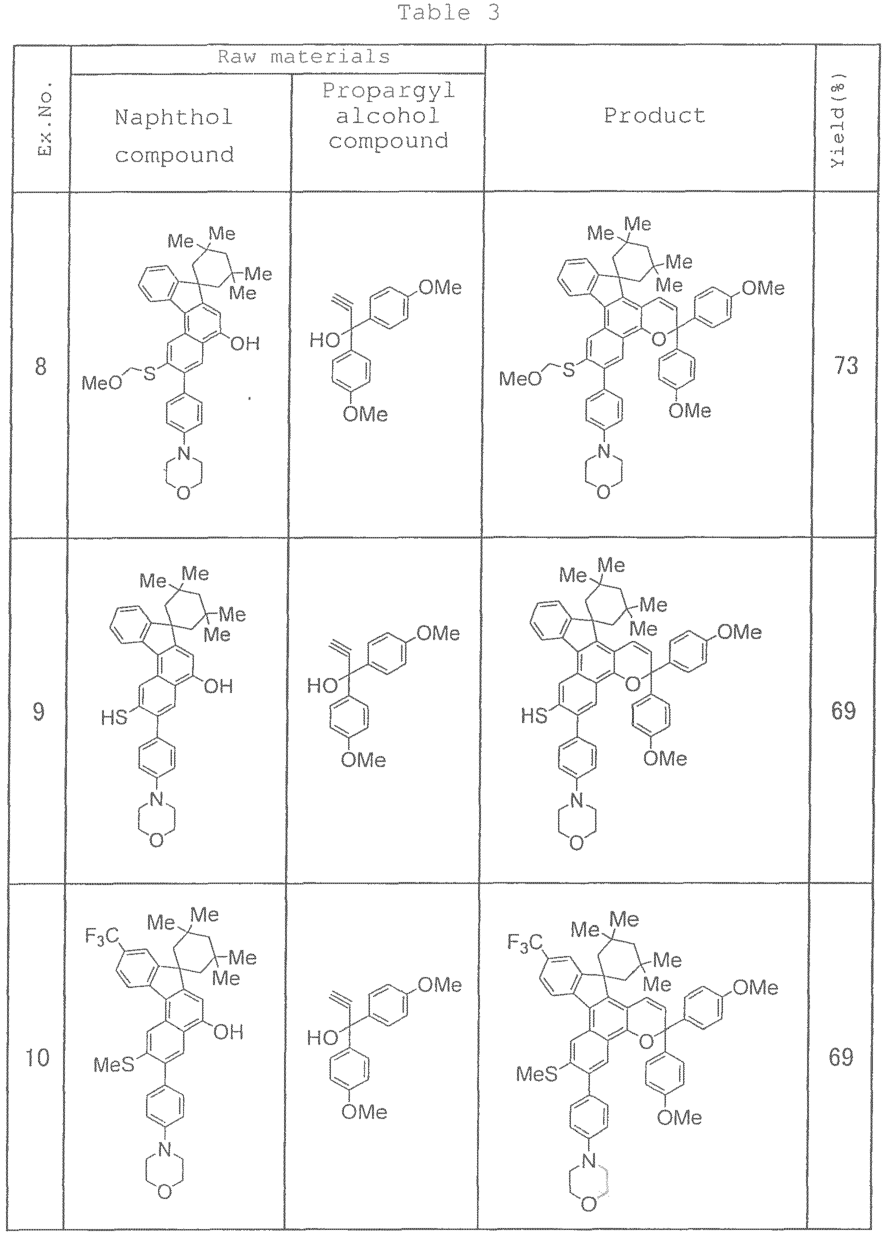

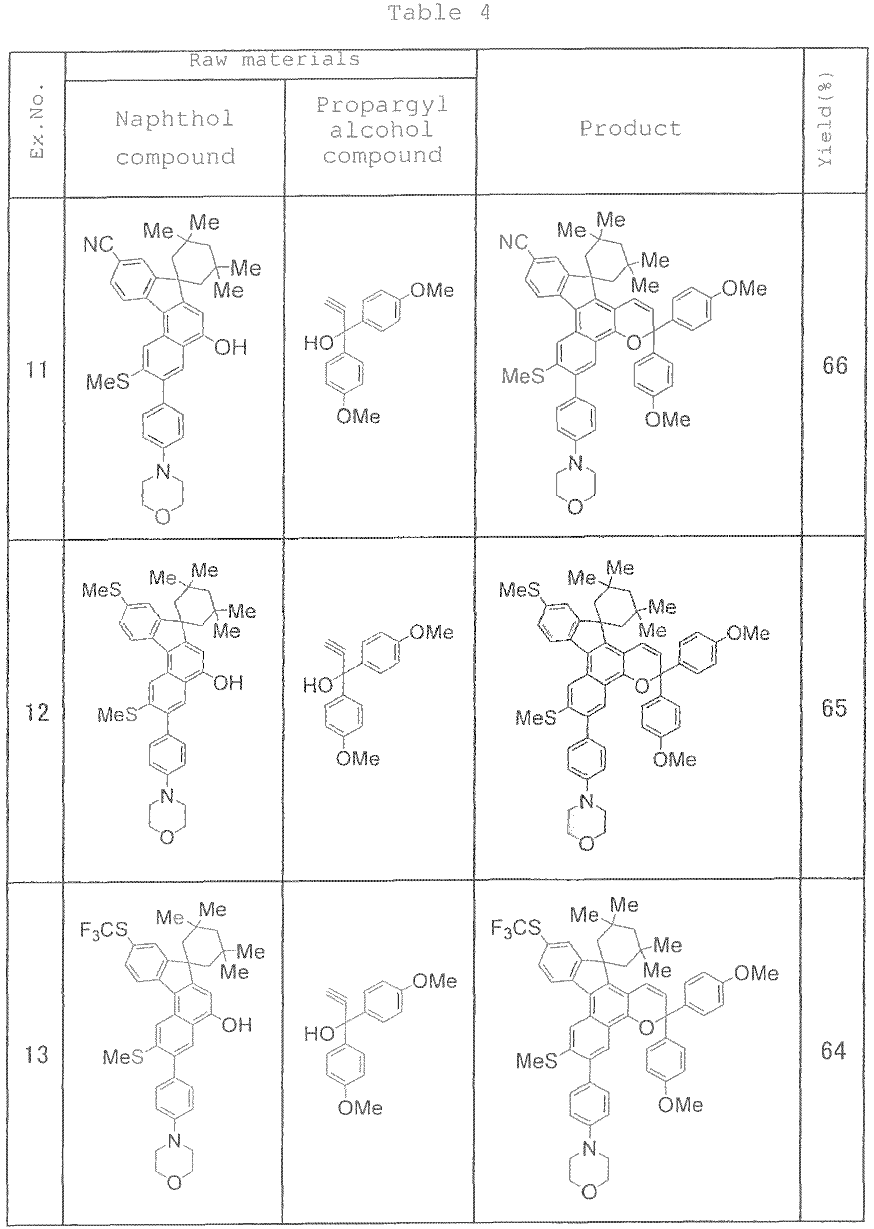

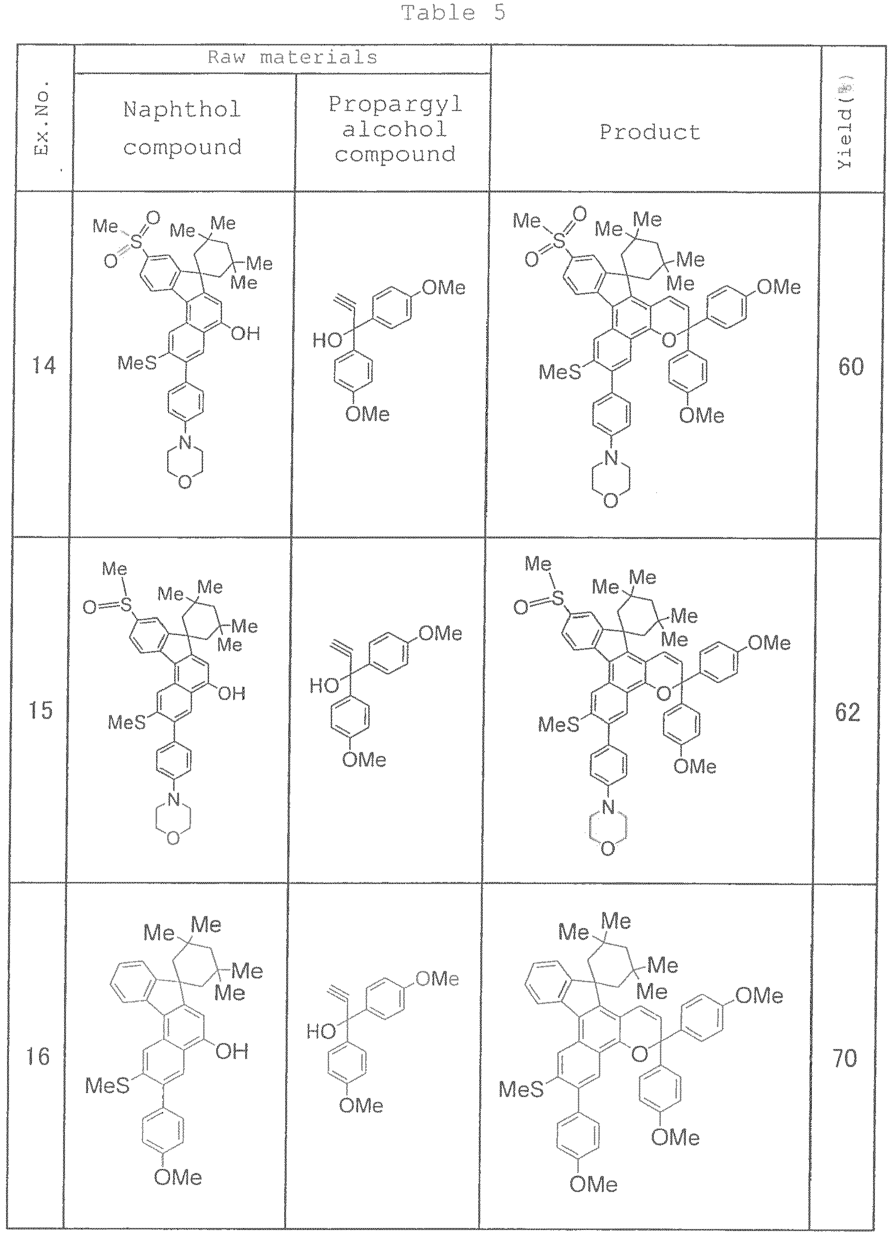

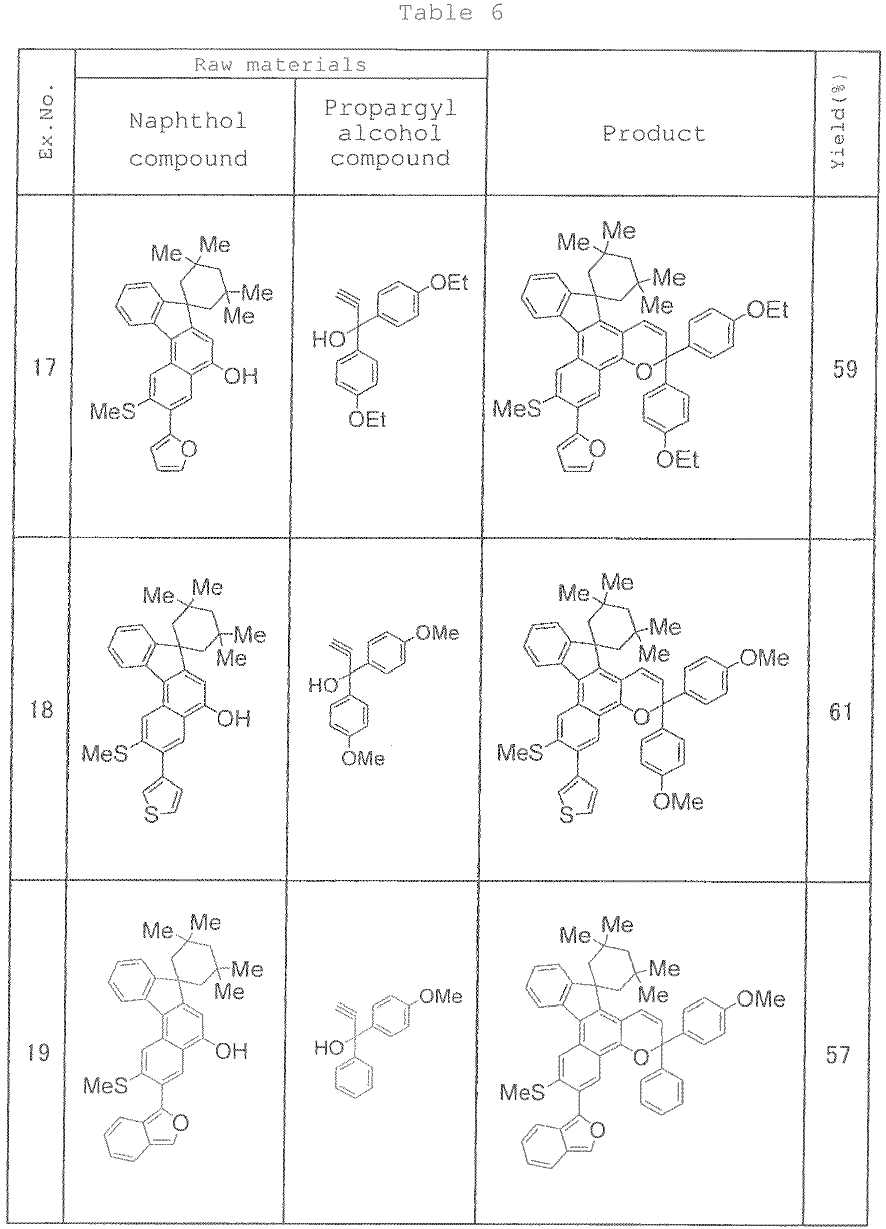

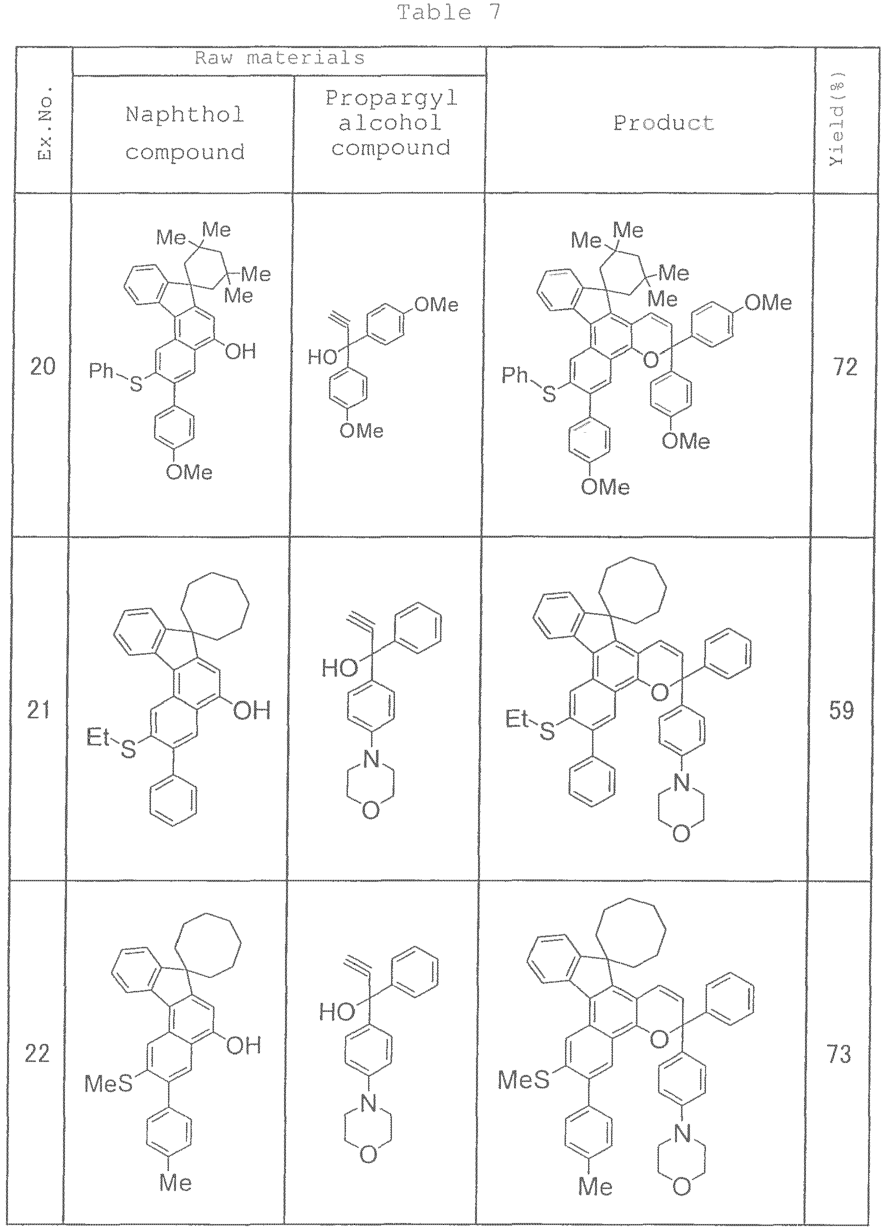

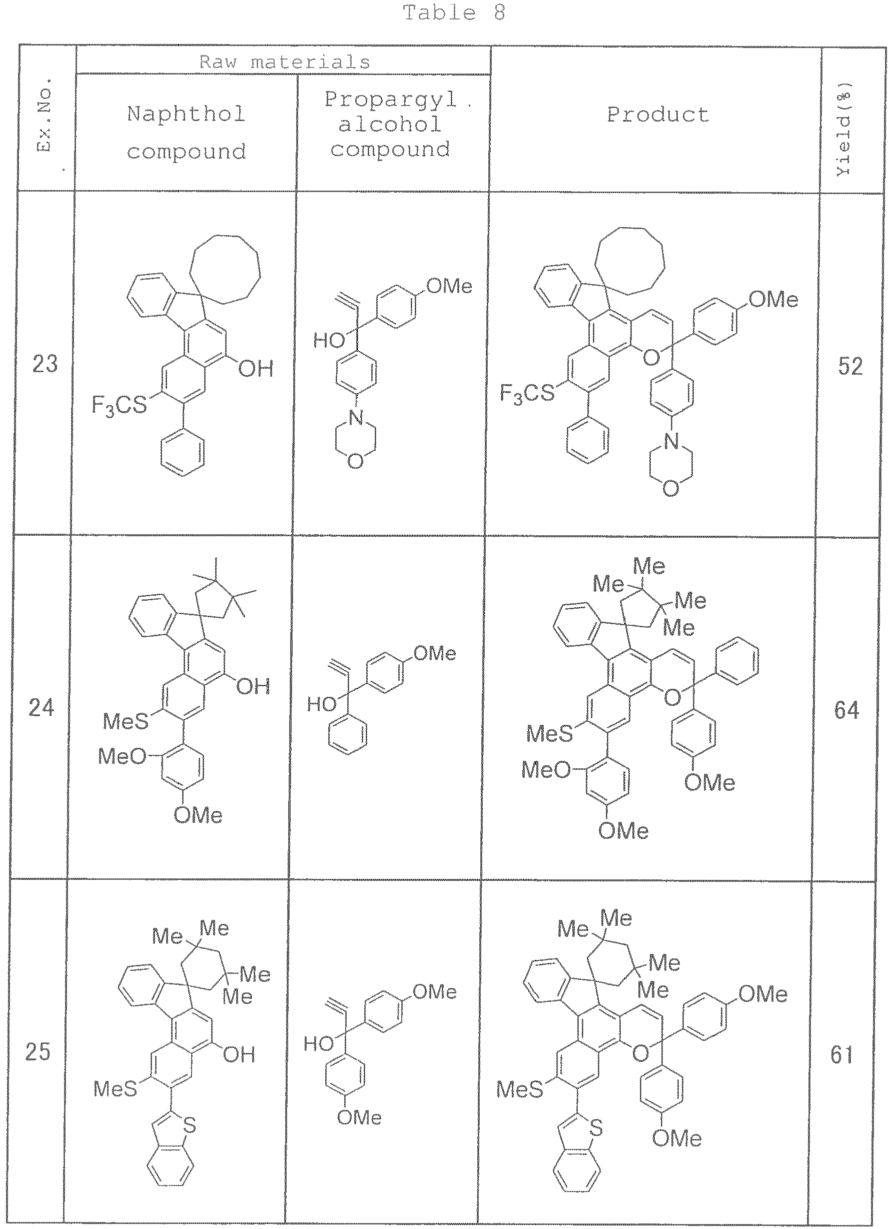

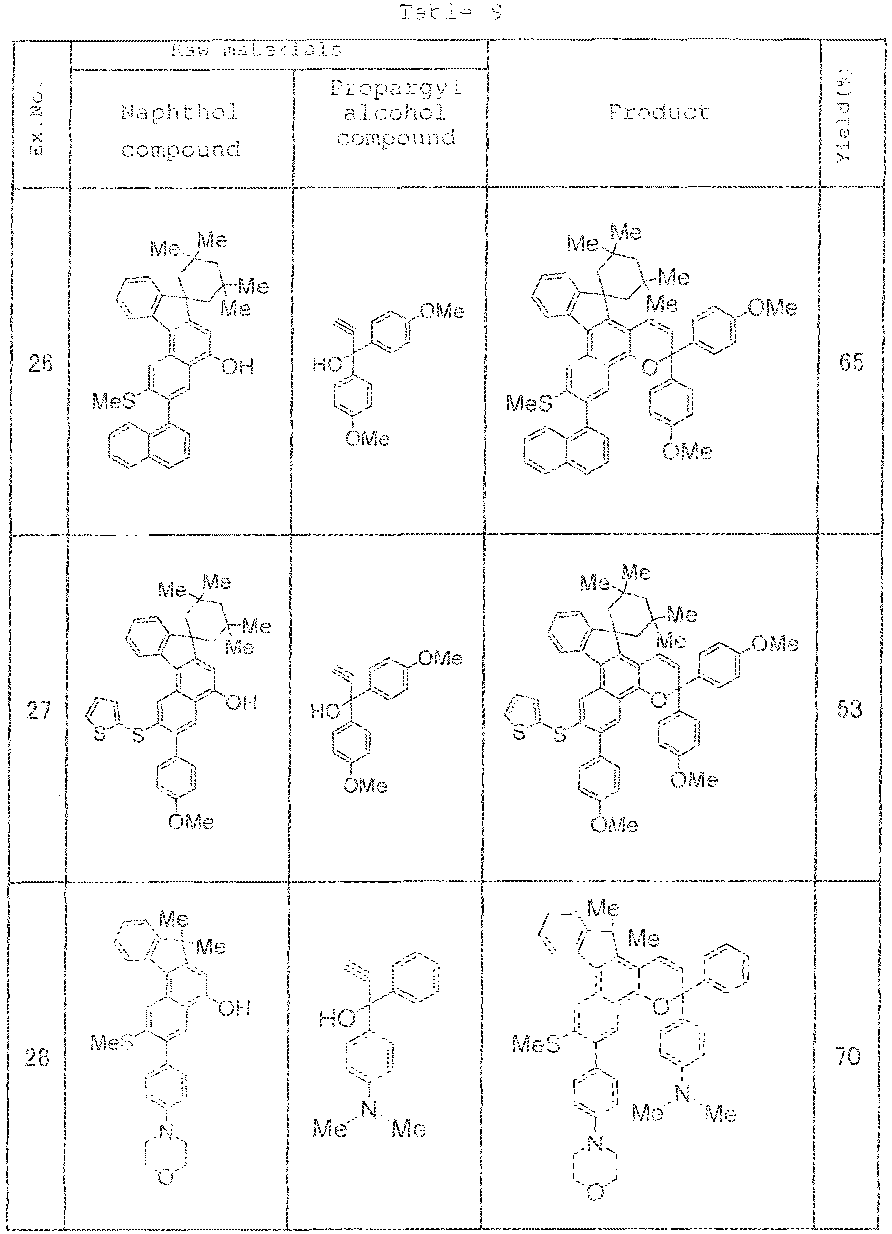

**Table 10**

| Example No. | Elemental analysis values | | | | | | | | ¹H-NMR (NMR) |
|---|---|---|---|---|---|---|---|---|---|
| | Experimental values | | | | Calculated values | | | | |
| | C | H | N | S | C | H | N | S | |
| 2 | 79.88 | 6.21 | 1.71 | 3.88 | 79.67 | 6.81 | 1.72 | 3.94 | δ 5.0-9.0 20H |
| | | | | | | | | | δ 0.5-4.5 35H |
| 3 | 80.74 | 6.77 | 1.78 | 4.12 | 80.90 | 6.92 | 1.81 | 4.15 | δ 5.0-9.0 20H |
| | | | | | | | | | δ 0.5-4.5 33H |
| 4 | 80.52 | 6.56 | 0.00 | 4.25 | 80.61 | 6.49 | 0.00 | 4.30 | δ 5.0-9.0 20H |
| | | | | | | | | | δ 0.5-4.5 28H |
| 5 | 81.72 | 6.89 | 0.00 | 3.79 | 81.98 | 6.52 | 0.00 | 3.84 | δ 5.0-9.0 25H |
| | | | | | | | | | δ 0.5-4.5 29H |
| 6 | 82.46 | 6.41 | 0.00 | 4.37 | 82.38 | 6.64 | 0.00 | 4.40 | δ 5.0-9.0 21H |
| | | | | | | | | | δ 0.5-4.5 27H |
| 7 | 80.36 | 7.46 | 1.59 | 3.61 | 80.33 | 7.20 | 1.59 | 3.63 | δ 5.0-9.0 20H |
| | | | | | | | | | δ 0.5-4.5 43H |
| 8 | 78.43 | 6.56 | 1.62 | 3.77 | 78.26 | 6.81 | 1.66 | 3.80 | δ 5.0-9.0 20H |
| | | | | | | | | | δ 0.5-4.5 37H |
| 9 | 79.54 | 6.91 | 1.71 | 4.06 | 79.57 | 6.68 | 1.75 | 4.01 | δ 5.0-9.0 20H |
| | | | | | | | | | δ 0.5-4.5 33H |
| 10 | 74.68 | 6.12 | 1.62 | 3.61 | 74.89 | 6.17 | 1.59 | 3.64 | δ 5.0-9.0 19H |
| | | | | | | | | | δ 0.5-4.5 35H |
| 11 | 78.91 | 6.35 | 3.31 | 3.81 | 78.73 | 6.49 | 3.34 | 3.82 | δ 5.0-9.0 19H |
| | | | | | | | | | δ 0.5-4.5 35H |
| 12 | 76.69 | 6.82 | 1.66 | 7.51 | 76.80 | 6.68 | 1.63 | 7.46 | δ 5.0-9.0 19H |
| | | | | | | | | | δ 0.5-4.5 38H |
| 13 | 72.43 | 5.77 | 1.56 | 7.08 | 72.26 | 5.95 | 1.53 | 7.02 | δ 5.0-9.0 19H |
| | | | | | | | | | δ 0.5-4.5 35H |
| 14 | 73.98 | 6.61 | 1.71 | 7.21 | 74.04 | 6.44 | 1.57 | 7.19 | δ 5.0-9.0 19H |
| | | | | | | | | | δ 0.5-4.5 38H |
| 15 | 75.49 | 6.71 | 1.55 | 7.40 | 75.39 | 6.56 | 1.60 | 7.32 | δ 5.0-9.0 19H |
| | | | | | | | | | δ 0.5-4.5 38H |

**Table 11**

| Example No. | Elemental analysis values | | | | | | | | ¹H-NMR (NMR) |
|---|---|---|---|---|---|---|---|---|---|
| | Experimental values | | | | Calculated values | | | | |
| | C | H | N | S | C | H | N | S | |
| 16 | 80.56 | 6.61 | | 4.27 | 80.70 | 6.64 | | 4.22 | δ 5.0-9.0 20H |
| | | | | | | | | | δ 0.5-4.5 35H |
| 17 | 80.51 | 7.97 | | 4.54 | 80.39 | 7.75 | | 4.29 | δ 5.0-9.0 20H |
| | | | | | | | | | δ 0.5-4.5 33H |
| 18 | 78.34 | 6.53 | | 8.97 | 78.44 | 6.31 | | 8.73 | δ 5.0-9.0 20H |
| | | | | | | | | | δ 0.5-9.5 28H |
| 19 | 82.74 | 6.17 | | 4.53 | 82.89 | 6.27 | | 4.34 | δ 5.0-9.0 25H |
| | | | | | | | | | δ 0.5-4.5 29H |
| 20 | 82.04 | 6.59 | | 3.67 | 81.92 | 6.38 | | 3.91 | δ 5.0-9.0 21H |
| | | | | | | | | | δ 0.5-4.5 27H |
| 21 | 82.57 | 6.82 | 2.09 | 4.45 | 82.78 | 6.67 | 1.89 | 4.33 | δ 5.0-9.0 20H |
| | | | | | | | | | δ 0.5-4.5 43H |
| 22 | 82.79 | 6.53 | 2.12 | 4.22 | 82.78 | 6.67 | 1.89 | 4.33 | δ 5.0-9.0 20H |
| | | | | | | | | | δ 0.5-4.5 37H |
| 23 | 75.94 | 6.00 | 1.80 | 3.66 | 75.80 | 5.87 | 1.70 | 3.89 | δ 5.0-9.0 20H |
| | | | | | | | | | δ 0.5-4.5 33H |
| 24 | 80.66 | 6.60 | | 4.33 | 80.61 | 6.49 | | 4.30 | δ 5.0-9.0 19H |
| | | | | | | | | | δ 0.5-4.5 35H |
| 25 | 79.34 | 6.11 | | 8.34 | 79.55 | 6.16 | | 8.17 | δ 5.0-9.0 19H |
| | | | | | | | | | δ 0.5-4.5 35H |
| 26 | 83.32 | 6.41 | | 4.27 | 83.25 | 6.47 | | 4.12 | δ 5.0-9.0 19H |
| | | | | | | | | | δ 0.5-9.5 38H |
| 27 | 78.40 | 6.01 | | 7.63 | 78.42 | 6.09 | | 7.75 | δ 5.0-9.0 19H |
| | | | | | | | | | δ 0.5-4.5 35H |
| 28 | 80.77 | 6.31 | 3.97 | 4.41 | 80.54 | 6.33 | 4.00 | 4.57 | δ 5.0-9.0 19H |
| | | | | | | | | | δ 0.5-4.5 38H |

In the chemical formulas, "Me" denotes a methyl group, "Et" an ethyl group, "Ph" a phenyl group and "Bn" a benzyl group through this text.

### Examples 29 to 56

### (evaluation of physical properties of photochromic plastic lens manufactured by coating method)

The chromene compound No. 1 obtained in Example 1 was mixed with a photopolymerization initiator and polymerizable monomers, the resulting mixture was applied to the surface of a lens substrate, and ultraviolet light was applied to polymerize the coating film on the surface of the lens substrate.

As for the photochromic curable composition, a mixture of 50 parts by mass of
2,2-bis(4-methacryloyloxypentaethoxyphenyl)propane, 10 parts by mass of polyethylene glycol diacrylate (average molecular weight of 532), 10 parts by mass of trimethylolpropane trimethacrylate, 10 parts by mass of polyester oligomer hexaacrylate (EB-1830 of Daicel UCB Co., Ltd.) and 10 parts by mass of glycidyl methacrylate as radically polymerizable monomers was used. After 1 part by mass of the chromene compound No. 1 obtained in Example 1 was added to and fully mixed with 90 parts by mass of the mixture of these radically polymerizable monomers, 0.3 part by mass of CGI1800 {a mixture of 1-hydroxycyclohexylphenyl ketone and bis(2,6-dimethoxybenzoyl)-2,4,4-trimethylpentylphosphine oxide (weight ratio of 3:1)} as a photopolymerization initiator, 5 parts by mass of bis(1,2,2,6,6-pentamethyl-4-piperidyl)sebacate and 3 parts by mass of
ethylenebis(oxyethylene)bis[3-(5-tert-butyl-4-hydroxy-m-tolyl)propionate] as a stabilizer, 7 parts by mass of γ-methacryloxypropyl trimethoxysilane as a silane coupling agent and 3 parts by mass of N-methyldiethanolamine were added to and fully mixed with the above mixture to obtain a photochromic curable composition.

Subsequently, about 2 g of the photochromic curable composition obtained by the above method was applied to the surface of a lens substrate (CR39: allyl resin plastic lens; refractive index of 1.50) by using the 1H-DX2 spin coater of MIKASA Co., Ltd. This coated lens was irradiated with light from a metal halide lamp having an output of 120 mW/cm² in a nitrogen gas atmosphere for 3 minutes to manufacture an optical article (photochromic plastic lens) which was covered with a cured polymer film containing the chromene compound dispersed therein (thickness of polymer film: 40 µm).

The following photochromic properties of the obtained photochromic plastic lens were evaluated. The results obtained by using the chromene compound of Example 1 are shown in Table 4.
[1] Maximum absorption wavelength, (λₘₐₓ): This is the maximum absorption wavelength after color development obtained by means of the spectrophotometer (MCPD3000 instantaneous multi-channel photodetector) of Otsuka Electronics Co., Ltd. and used as an index of color at the time of color development.
[2] Color optical density (A₀) : This is the difference between absorbance {ε(120)} after 120 seconds of exposure at the above maximum absorption wavelength and absorbance ε(0) under no exposure and used as an index of color optical density. It can be said that as this value becomes larger, photochromic properties become better.
[3] Double peak characteristic (A_{Y}/A_{B}) : This is the ratio of color optical density (A_{Y}: value of λₘₐₓ) at a yellow range (absorption peak having a maximum absorption wavelength at 430 to 530 nm) and color optical density (A_{B}: value of λₘₐₓ) at a blue range (absorption peak having a maximum absorption wavelength at 550 to 650 nm) and used as an index of double peak characteristic.
[4] Fading half period [τ1/2] (sec.)] : time required for the reduction of the absorbance at the above maximum absorption wavelength of a sample to 1/2 of {ε(120)-ε(0)} when exposure is stopped after 120 seconds of exposure and used as an index of fading speed. As this time becomes shorter, the fading speed becomes higher.
[5] Absorption end {λ₀} : After the photochromic plastic lens obtained under the above conditions is used as a sample and kept in the dark for one day, the ultraviolet light transmittance (T%) at 300 to 800 nm of the sample is measured with an ultraviolet visible spectrophotometer (UV-2550 of Shimadzu Corporation) at room temperature. A tangent line is drawn on the obtained ultraviolet light absorption curve to ensure that the transmittance (T%) of the ultraviolet light absorption curve passes a point of 50 % so as to obtain an absorption wavelength at which the transmittance (T%) of the tangent line becomes 0 as the absorption end (absorption end of the ultraviolet light spectrum) and used as an index of initial coloration. For example, in an optical article such as a spectacle lens, as this value becomes smaller, initial coloration becomes weaker and transparency under no exposure becomes higher.
[6] Thermochromism {T₀} : The photochromic plastic lens obtained under the above conditions is used as a sample and its transmittance (T%) at 300 to 800 nm is measured with an ultraviolet visible spectrophotometer (UV-2550 of Shimadzu Corporation) at room temperature. This is a transmittance at a wavelength at which the transmittance at 430 to 650 nm becomes minimal and used as an index of initial coloration. As this value becomes larger, initial coloration becomes weaker and transparency under no exposure becomes higher.
[7] Residual rate (A₅₀/A₀ x 100): A deterioration promotion test is made on the obtained photochromic plastic lens by using the X25 xenon weather meter of Suga Test Instruments Co., Ltd. for 50 hours. Thereafter, the above color optical density is evaluated before and after the test by measuring the color optical density (A₀) before the test and the color optical density (A₅₀) after the test in order to obtain the ratio (A₅₀/A₀) of these values as residual rate which is used as an index of color development durability. As the residual rate becomes higher, color development durability becomes higher.

Photochromic plastic lenses were obtained and their photochromic properties were evaluated in the same manner as described above except that the chromene compounds (Nos. 2 to 28) obtained in Examples 2 to 28 were used. The results are shown in Tables 12 to 13.

**Table 12**

| Example No. | Chromene compound No.* | λMax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A₅₀/A₀) X 100 (%) |
| 29 | 1 | 454 | 0.53 | 1.20 | 40 | 412 | 84 | 84 |
| | | 571 | 0.44 | | 41 | | 83 | 84 |
| 30 | 2 | 457 | 0.58 | 1.26 | 42 | 415 | 83 | 84 |
| | | 575 | 0.46 | | 43 | | 83 | 84 |
| 31 | 3 | 461 | 0.62 | 1.35 | 43 | 415 | 82 | 84 |
| | | 579 | 0.46 | | 43 | | 83 | 84 |
| 32 | 4 | 455 | 0.56 | 1.22 | 50 | 416 | 83 | 80 |
| | | 572 | 0.46 | | 51 | | 83 | 80 |
| 33 | 5 | 454 | 0.52 | 1.18 | 52 | 415 | 83 | 80 |
| | | 572 | 0.44 | | 51 | | 83 | 80 |
| 34 | 6 | 451 | 0.45 | 1.02 | 38 | 408 | 84 | 82 |
| | | 571 | 0.44 | | 38 | | 83 | 82 |
| 35 | 7 | 457 | 0.60 | 1.28 | 46 | 416 | 84 | 84 |
| | | 579 | 0.47 | | 46 | | 84 | 83 |
| 36 | 8 | 454 | 0.52 | 1.18 | 52 | 415 | 82 | 84 |
| | | 573 | 0.44 | | 53 | | 82 | 84 |
| 37 | 9 | 443 | 0.34 | 1.10 | 36 | 415 | 83 | 84 |
| | | 562 | 0.31 | | 35 | | 83 | 83 |
| 38 | 10 | 459 | 0.41 | 1.27 | 32 | 414 | 84 | 82 |
| | | 575 | 0.32 | | 32 | | 83 | 82 |
| 39 | 11 | 461 | 0.39 | 1.30 | 29 | 414 | 83 | 81 |
| | | 579 | 0.30 | | 30 | | 83 | 82 |
| 40 | 12 | 456 | 0.56 | 1.51 | 34 | 419 | 83 | 83 |
| | | 575 | 0.37 | | 34 | | 83 | 83 |
| 41 | 13 | 458 | 0.57 | 1.63 | 34 | 420 | 83 | 83 |
| | | 571 | 0.35 | | 33 | | 83 | 83 |
| 42 | 14 | 458 | 0.55 | 1.49 | 33 | 419 | 82 | 80 |
| | | 570 | 0.37 | | 33 | | 83 | 81 |
| 43 | 15 | 458 | 0.54 | 1.46 | 33 | 418 | 82 | 81 |
| | | 571 | 0.37 | | 33 | | 82 | 81 |

| | | | | | | | | |
|---|---|---|---|---|---|---|---|---|
| *Example No. of chromene compound used in Examples | | | | | | | | |

**Table 13**

| Example No. | Chromene compound No.* | λMax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A₅₀/A₀) X 100 (%) |
| 44 | 16 | 452 | 0.50 | 1.20 | 39 | 410 | 86 | 83 |
| | | 569 | 0.42 | | 39 | | 86 | 83 |
| 45 | 17 | 448 | 0.32 | 1.12 | 49 | 409 | 84 | 73 |
| | | 568 | 0.29 | | 49 | | 84 | 73 |
| 46 | 18 | 450 | 0.41 | 1.09 | 40 | 408 | 83 | 71 |
| | | 569 | 0.38 | | 40 | | 83 | 71 |
| 47 | 19 | 443 | 0.50 | 0.35 | 55 | 412 | 81 | 72 |
| | | 561 | 0.37 | | 55 | | 81 | 72 |
| 48 | 20 | 459 | 0.52 | 1.02 | 58 | 419 | 81 | 81 |
| | | 480 | 0.51 | | 58 | | 81 | 81 |
| 49 | 21 | 471 | 0.71 | 0.99 | 73 | 410 | 80 | 82 |
| | | 586 | 0.72 | | 73 | | 80 | 82 |
| 50 | 22 | 475 | 0.69 | 1.02 | 75 | 410 | 81 | 82 |
| | | 588 | 0.68 | | 75 | | 81 | 82 |
| 51 | 23 | 475 | 0.59 | 1.20 | 61 | 420 | 87 | 83 |
| | | 589 | 0.49 | | 61 | | 87 | 83 |
| 52 | 24 | 447 | 0.63 | 1.59 | 59 | 411 | 82 | 72 |
| | | 562 | 0.40 | | 59 | | 85 | 72 |
| 53 | 25 | 458 | 0.42 | 1.21 | 46 | 418 | 83 | 81 |
| | | 576 | 0.35 | | 46 | | 83 | 81 |
| 54 | 26 | 453 | 0.57 | 1.19 | 51 | 416 | 83 | 80 |
| | | 580 | 0.48 | | 51 | | 83 | 80 |
| 55 | 27 | 462 | 0.54 | 1.05 | 60 | 420 | 81 | 78 |
| | | 583 | 0.52 | | 60 | | 81 | 78 |
| 56 | 28 | 479 | 0.72 | 1.09 | 72 | 412 | 80 | 80 |
| | | 592 | 0.66 | | 72 | | 80 | 80 |

| | | | | | | | | |
|---|---|---|---|---|---|---|---|---|
| * Example No. of chromene compound used in Examples | | | | | | | | |

Comparative Examples 1 to 3

For comparison, photochromic plastic lenses were obtained and their characteristic properties were evaluated in the same manner as in Examples except that compounds represented by the following formulas (A) to (C) were used. The results are shown in Table 14.

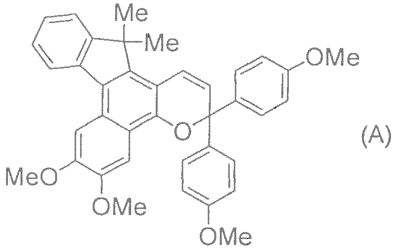

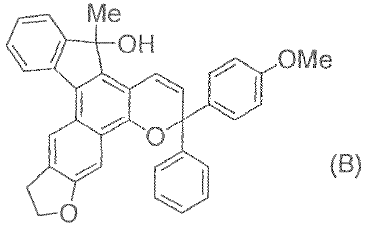

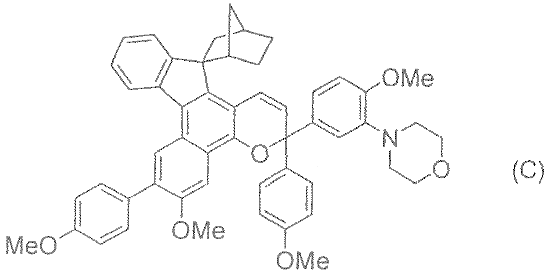

**Table 14**

| Comparative Example No. | Compound No. | λMax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A₅₀/A₀) X 100 (%) |
| 1 | (A) | 457 | 0.69 | 1.56 | 195 | 397 | 67 | 76 |
| | | 574 | 0.45 | | 196 | | 75 | 77 |
| 2 | (B) | 455 | 0.30 | 0.94 | 83 | 410 | 77 | 35 |
| | | 576 | 0.32 | | 83 | | 78 | 35 |
| 3 | (C) | 458 | 0.44 | 1.20 | 68 | 422 | 84 | 85 |
| | | 568 | 0.37 | | 68 | | 86 | 84 |

The chromene compound of the present invention having high double peak characteristic (A_{Y}/A_{B}) is preferred because a blue compound having excellent durability can be used for delicate color control. For example, A_{Y}/A_{B} is preferably not less than 0.80, more preferably not less than 1.00, much more preferably not less than 1.10, particularly preferably not less than 1.20, most preferably not less than 1.30. The upper limit value of A_{Y}/A_{B} is 2.00 in consideration of the chromene compound of the present invention.

The fading half period τ1/2 is preferably 50 seconds or more to less than 130 seconds, more preferably 50 seconds or more to less than 100 seconds, particularly preferably 50 seconds or more to less than 80 seconds.

The transmittance by thermochromism is preferably not less than 80 %, more preferably not less than 83 %, particularly preferably not less than 85 %.

The absorption end is existent at preferably 400 to 420 nm, more preferably 405 to 420 nm from the viewpoints of initial coloration and color development sensitivity.

It is understood that the photochromic plastic lenses of Examples 29 to 56 which were obtained from the chromene compounds of the present invention are superior in color optical density, fading speed and durability to photochromic plastic lenses of Comparative Example 1 (chromene compound represented by the above formula (A)), Comparative Example 2 (chromene compound represented by the above formula (B)) and Comparative Example 3 (chromene compound represented by the above formula (C)) while having high double peak characteristic.

The photochromic plastic lenses of Comparative Examples 1 and 2 have strong initial coloration by thermochromism. The photochromic plastic lens of Comparative Example 3 has strong initial coloration because its absorption end goes beyond 420 nm into the visible range. In contrast to this, Examples of the present invention have weak initial coloration as thermochromism is small and the absorption end exists at a short wavelength range.

Examples of the naphthol compound are given below.

### Example 57

80.0 g (400.0 mmol) of a benzene compound represented by the above formula (9) was added dropwise to a dichloromethane solution (400 ml) containing 64.0 g (480.0 mmol) of aluminum chloride and 56.2 g (400.0 mmol) of benzoyl chloride cooled to 0°C. After addition, the resulting mixture was stirred for 2 hours. After a reaction, the reaction product was washed with water, the solvent was removed, and the obtained product was purified by column chromatography to obtain a benzophenone derivative represented by the following formula (20) as 74.4 g (244.1 mmol, yield of 61 %) of a yellow solid.

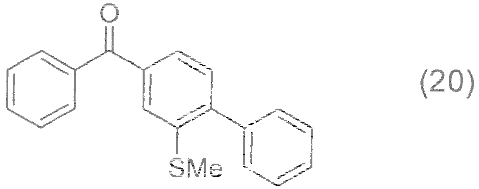

The benzophenone derivative of the above formula (20) and 48.9 g (280.8 mmol) of diethyl succinate were dissolved in 300 ml of tetrahydrofuran and heated at 55°C. A tetrahydrofuran solution (300 ml) containing 31.5 g (280.8 mmol) of potassium-t-butoxide was added dropwise to this solution and stirred for 1 hour. After a reaction, the reaction product was washed with concentrated hydrochloric acid and then with water, and the solvent was removed to obtain a compound represented by the following formula (21) as 105. 4 g (244.1 mmol, yield of 100 %) of an orange oil.

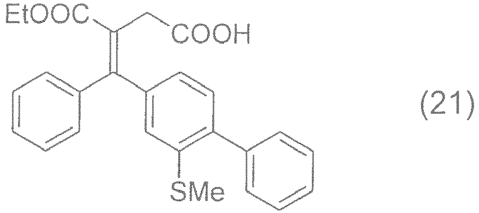

The compound of the above formula (21), 20.0 g (244.1 mmol) of sodium acetate and 125.7 g (1220.5 mmol) of acetic anhydride were dissolved in 400 ml of toluene and refluxed for 4 hours. After a reaction, the reaction product was washed with water, the solvent was removed, and the obtained product was purified by recrystallization with methanol so as to obtain a compound represented by the following formula (22) as 21.2 g (46.4 mmol, yield of 19 %) of an orange solid.

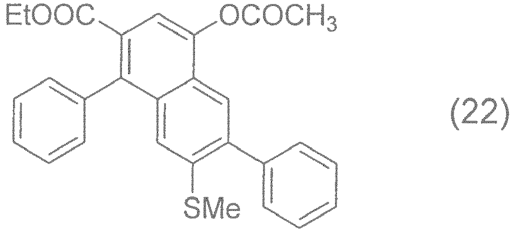

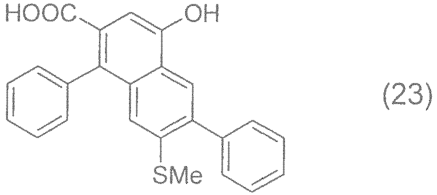

The compound of the above formula (22) was dispersed into 100 ml of methanol. 185 ml of an aqueous solution containing 18.5 g (464.0 mmol) of sodium hydroxide was added to this solution and refluxed for 3 hours. After a reaction, the reaction product was washed with concentrated hydrochloric acid and then with water, the solvent was removed, and the obtained product was purified by reslurrying with toluene to obtain a carboxylic acid derivative represented by the above formula (23) as 16.8 g (43.6 mmol, yield of 94 %) of a yellow solid.

The carboxylic acid derivative of the above formula (23) and 13.2 g (95.9 mmol) of benzyl chloride were dissolved in 120 ml of N,N-dimethylformamide. 13.7 g (109.0 mmol) of potassium carbonate was added to this solution, and the resulting mixture was heated to 60°C and stirred for 3 hours. After a reaction, the reaction product was washed with water, and the solvent was removed to obtain a compound represented by the following formula (24) as 22.5 g (39.7 mmol, yield of 91 %) of a yellow oil.

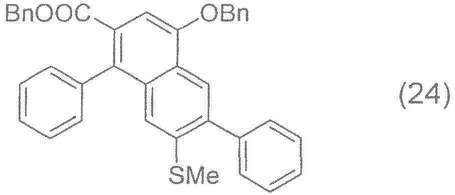

The compound of the above formula (24) was dispersed into 400 ml of isopropyl alcohol. 143 ml of an aqueous solution containing 28.6 g (715.0 mmol) of sodium hydroxide was added to this solution and refluxed for 4 hours. After a reaction, the reaction product was washed with concentrated hydrochloric acid and then with water, the solvent was removed, and the obtained product was purified by reslurrying with toluene to obtain a carboxylic acid derivative represented by the following formula (25) as 18.3 g (38.5 mmol, yield of 97 %) of a yellow solid.

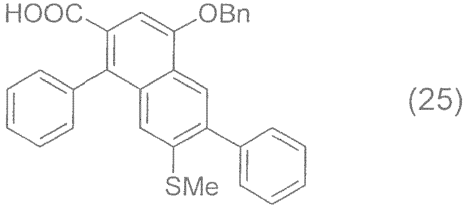

The carboxylic acid derivative of the above formula (25) was dispersed into 400 ml of toluene. 77.8 g (770.0 mmol) of triethylamine and 13.7 g (50.0 mmol) of diphenylphosphorylazide were added to this solution and stirred at room temperature for 2 hours. 20.0 g (435.3 mmol) of ethanol was added to this solution to carry out a reaction at 70 °C for 2 hours. 500 ml of ethanol was added to this solution, and then 64.6 g (1155.0 mmol) of potassium hydroxide was added and refluxed for 4 hours. After a reaction, ethanol was distilled off at normal pressure, tetrahydrofuran was added, the reaction solution was washed with water, and the solvent was removed to obtain a compound represented by the following formula (26) as 13.9 g (31.2 mmol, yield of 81 %) of a yellow solid.

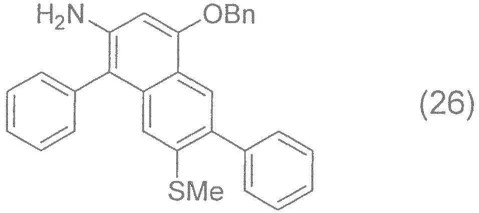

The compound of the above formula (26) was dispersed into 400 ml of acetonitrile, and 93.8 g (154.4 mmol) of a 6 % hydrochloric acid aqueous solution was added and cooled to 0 to 5 °C. 9.7 g (46.8 mmol) of a 33 % sodium nitrite aqueous solution was added to this solution and stirred for 30 minutes. 26.0 g (156.0 mmol) of a 50 % potassium iodide aqueous solution was added to this solution and stirred at room temperature for 4 hours. After a reaction, toluene was added, the reaction solution was washed with water, the solvent was removed, and the obtained product was purified by column chromatography to obtain a compound represented by the following formula (27) as 12.9 g (23.1 mmol, yield of 74 %) of a yellow solid.

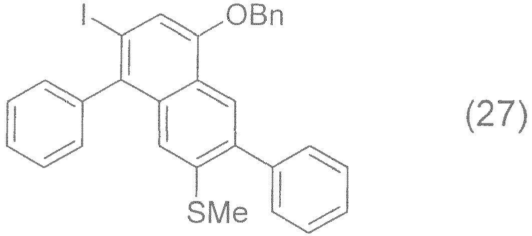

The compound of the above formula (27) was dispersed into 400 ml of toluene and cooled to -30°C. 21.6 ml (34.6 mmol) of n-butyl lithium (1.6 M hexane solution) was added dropwise to this solution and stirred for 30 minutes. 11.4 g of a toluene solution containing 5.7 g (37.0 mmol) of 3,3,5, 5-tetramethylcyclohexanone was added dropwise to this solution and stirred at 0°C for 1 hour. After a reaction, toluene was added, the reaction solution was washed with water, the solvent was removed, and the obtained product was purified by reslurrying with methanol to obtain a compound represented by the following formula (28) as 8.4 g (14.3 mmol, yield of 62 %) of a yellow solid.

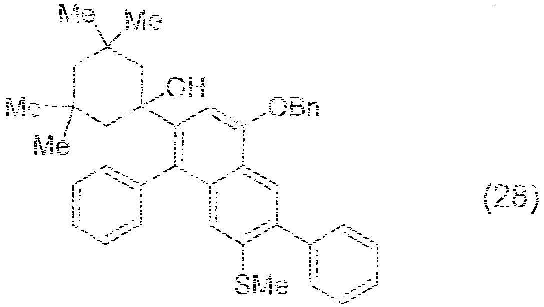

The compound of the above formula (28) and 103.2 mg (0.42 mmol) of (±)-10-camphorsulfonic acid were dissolved in 150 ml of toluene and refluxed for 30 minutes. After the obtained solution was left to be cooled to room temperature, this solution was added to 70 ml of a toluene solution containing 3.5 g (21. 5 mmol) of p-toluenesulfonic acid heated at 90°C and refluxed for 6 hours. After a reaction, the reaction product was washed with water, the solvent was removed, and the obtained product was purified by column chromatography to obtain a naphthol compound represented by the following formula (29) as 2.8 g (5.9 mmol, yield of 41 %) of a yellow solid.

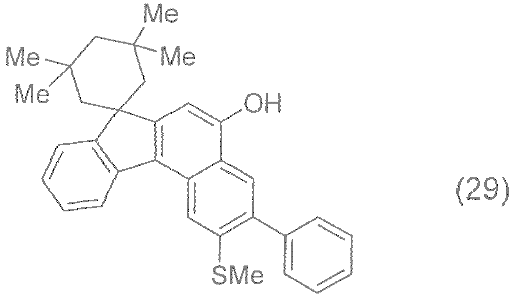

The elemental analysis values of this product were 82.69 % of C, 7.39 % of H and 6.79 % of S which were almost equal to the calculated values of C₃₃H₃₄OS (C: 82.80 %, H: 7.16 %, S: 6.70 %).

When the proton nuclear magnetic resonance spectrum of the product was measured, it showed a 21H peak based on an alkyl group at δ of around 0.5 to 4.5 ppm and 13H peaks based on a hydroxyl group and an aromatic proton at 8 of around 5.0 to 9.0 ppm.

Further, when the ¹³C-nuclear magnetic resonance spectrum was measured, it showed a peak based on the carbon of an aromatic ring at δ of around 110 to 160 ppm and a peak based on the carbon of an alkyl group at δ of around 20 to 80 ppm.

It was confirmed from these results that the isolated product was a compound represented by the above formula (29) .

This compound is a naphthol compound used in the above Example 6.

### Examples 58 to 83

The naphthol compound of Example 1 (naphthol compound of the above formula (17)) and naphthol compounds shown in the Tables 1 to 9 were synthesized in the same manner as in Example 57. When the structures of the obtained products were analyzed by using the same structure confirming means as in Example 57, it was confirmed that they were the naphthol compound of Example 1 (naphthol compound of the above formula (17)) and naphthol compounds used in Examples shown in Tables 1 to 9. Tables 15 and 16 show the elemental analysis values, calculated values obtained from the structural formulas of the compounds and characteristic ¹H-NMR spectra of these compounds.

**Table 15**

| Example No. | Chromene compound No.* | Elemental analysis values | | | | | | | | ¹H-NMR (NMR) |
|---|---|---|---|---|---|---|---|---|---|---|
| | | Experimental values | | | | Calculated values | | | | |
| | | C | H | N | S | C | H | N | S | |
| 57 | 1 | 78.14 | 7.05 | 0.00 | 6.01 | 78.03 | 7.11 | 0.00 | 5.95 | δ 5.0-9.0 11H |
| | | | | | | | | | | δ 0.5-4.5 27H |
| 58 | 2 | 79.01 | 7.24 | 2.56 | 5.72 | 78.82 | 7.33 | 2.48 | 5.69 | δ 5.0-9.0 12H |
| | | | | | | | | | | δ 0.5-4.5 29H |
| 59 | 3 | 80.55 | 7.67 | 2.77 | 6.09 | 80.57 | 7.53 | 2.68 | 6.15 | δ 5.0-9.0 12H |
| | | | | | | | | | | δ 0.5-4.5 27H |
| 60 | 4 | 80.24 | 6.99 | 0.00 | 6.54 | 80.12 | 6.93 | 0.00 | 6.48 | δ 5.0-9.0 13H |
| | | | | | | | | | | δ 0.5-4.5 21H |
| 61 | 5 | 82.24 | 6.91 | 0.00 | 5.51 | 82.15 | 6.89 | 0.00 | 5.48 | δ 5.0-9.0 19H |
| | | | | | | | | | | δ 0.5-4.5 21H |
| 62 | 7 | 79.92 | 7.73 | 2.34 | 5.12 | 79.83 | 7.82 | 2.22 | 5.07 | δ 5.0-9.0 12H |
| | | | | | | | | | | δ 0.5-4.5 37H |
| 63 | 8 | 76.82 | 7.20 | 2.33 | 5.43 | 76.86 | 7.30 | 2.36 | 5.40 | δ 5.0-9.0 12H |
| | | | | | | | | | | δ 0.5-4.5 31H |
| 64 | 9 | 78.77 | 7.11 | 2.51 | 5.91 | 78.65 | 7.15 | 2.55 | 5.83 | δ 5.0-9.0 12H |
| | | | | | | | | | | δ 0.5-4.5 27H |
| 65 | 10 | 72.20 | 6.43 | 2.31 | 5.12 | 72.24 | 6.38 | 2.22 | 5.08 | δ 5.0-9.0 11H |
| | | | | | | | | | | δ 0.5-4.5 29H |
| 66 | 11 | 77.49 | 6.97 | 4.61 | 5.44 | 77.51 | 6.85 | 4.76 | 5.45 | δ 5.0-9.0 11H |
| | | | | | | | | | | δ 0.5-4.5 29H |
| 67 | 12 | 74.92 | 7.02 | 2.16 | 10.62 | 74.84 | 7.11 | 2.30 | 10.52 | δ 5.0-9.0 11H |
| | | | | | | | | | | δ 0.5-4.5 32H |
| 68 | 13 | 68.89 | 6.01 | 2.04 | 9.71 | 68.75 | 6.07 | 2.11 | 9.66 | δ 5.0-9.0 11H |
| | | | | | | | | | | δ 0.5-4.5 29H |
| 69 | 14 | 71.04 | 6.78 | 2.09 | 10.06 | 71.10 | 6.75 | 2.18 | 9.99 | δ 5.0-9.0 11H |
| | | | | | | | | | | δ 0.5-4.5 32H |
| 70 | 15 | 72.84 | 6.99 | 2.25 | 10.24 | 72.92 | 6.92 | 2.24 | 10.25 | δ 5.0-9.0 11H |
| | | | | | | | | | | δ 0.5-4.5 32H |

| | | | | | | | | | | |
|---|---|---|---|---|---|---|---|---|---|---|
| *Example No. of chromene compound used in Examples | | | | | | | | | | |

**Table 16**

| Example No. | Chromene compound No.* | Elemental analysis values | | | | | | | | ¹H-NMR (NMR) |
|---|---|---|---|---|---|---|---|---|---|---|
| | | Experimental values | | | | Calculated values | | | | |
| | | C | H | N | S | C | H | N | S | |
| 71 | 16 | 80.52 | 7.29 | | 6.16 | 80.27 | 7.13 | | 6.30 | δ 5.0-9.0 11H |
| | | | | | | | | | | δ 0.5-4.5 27H |
| 72 | 17 | 79.28 | 6.89 | | 7.06 | 79.45 | 6.88 | | 6.84 | δ 5.0-9.0 12H |
| | | | | | | | | | | δ 0.5-4.5 29H |
| 73 | 18 | 76.95 | 6.65 | | 13.22 | 76.81 | 6.65 | | 13.23 | δ 5.0-9.0 12H |
| | | | | | | | | | | δ 0.5-4.5 27H |
| 74 | 19 | 81.05 | 6.71 | | 6.02 | 81.04 | 6.61 | | 6.18 | δ 5.0-9.0 13H |
| | | | | | | | | | | δ 0.5-4.5 21H |
| 75 | 20 | 82.18 | 6.75 | | 5.63 | 82.07 | 6.71 | | 5.62 | δ 5.0-9.0 19H |
| | | | | | | | | | | δ 0.5-4.5 21H |
| 76 | 21 | 82.82 | 7.03 | | 6.90 | 82.71 | 6.94 | | 6.90 | δ 5.0-9.0 12H |
| | | | | | | | | | | δ 0.5-4.5 37H |
| 77 | 22 | 82.94 | 6.99 | | 6.66 | 82.71 | 6.94 | | 6.90 | δ 5.0-9.0 12H |
| | | | | | | | | | | δ 0.5-4.5 31H |
| 78 | 23 | 74.32 | 5.53 | | 6.18 | 74.11 | 5.64 | | 6.18 | δ 5.0-9.0 12H |
| | | | | | | | | | | δ 0.5-4.5 27H |
| 79 | 24 | 77.88 | 6.68 | | 6.00 | 77.83 | 6.92 | | 6.11 | δ 5.0-9.0 11H |
| | | | | | | | | | | δ 0.5-4.5 29H |
| 80 | 25 | 78.61 | 6.45 | | 12.14 | 78.61 | 6.41 | | 11.99 | δ 5.0-9.0 11H |
| | | | | | | | | | | δ 0.5-4.5 29H |
| 81 | 26 | 83.93 | 6.80 | | 6.06 | 84.06 | 6.86 | | 6.06 | δ 5.0-9.0 11H |
| | | | | | | | | | | δ 0.5-4.5 32H |
| 82 | 27 | 76.99 | 6.19 | | 11.30 | 77.04 | 6.29 | | 11.12 | δ 5.0-9.0 11H |
| | | | | | | | | | | δ 0.5-4.5 29H |
| 83 | 28 | 77.02 | 6.35 | 3.05 | 6.65 | 77.05 | 6.25 | 3.00 | 6.86 | δ 5.0-9.0 11H |
| | | | | | | | | | | δ 0.5-4.5 32H |

| | | | | | | | | | | |
|---|---|---|---|---|---|---|---|---|---|---|
| *Example No. of chromene compound used in Examples | | | | | | | | | | |

### Effect of the Invention

The chromene compound of the present invention develops a color of a neutral tint and has little initial coloration, high color development sensitivity, high color optical density and high fading speed even when it is dispersed into a solution or a polymer solid matrix as well as excellent durability.

Therefore, when a photochromic lens is manufactured by using the chromene compound of the present invention, it develops a dark color of a neutral tint swiftly when it moves outside and returns to its original color swiftly when it returns inside from outside and has such high durability that it can be used for a long time.

## Claims

1. A chromene compound having a skeleton represented by the following formula (1) : 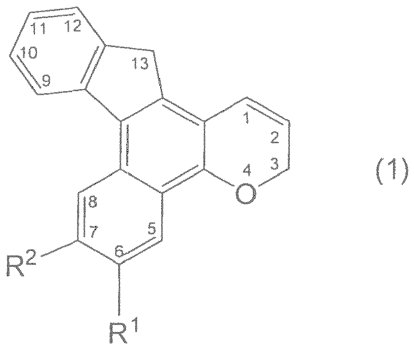 wherein R¹ is an aryl group or a heteroaryl group, and R² is a sulfur-containing substituent selected from the group consisting of thiol group, alkylthio group, alkoxyalkylthio group, haloalkylthio group, cycloalkylthio group, arylthio group and heteroarylthio group.

2. The chromene compound according to claim 1 which is represented by the following formula (2): 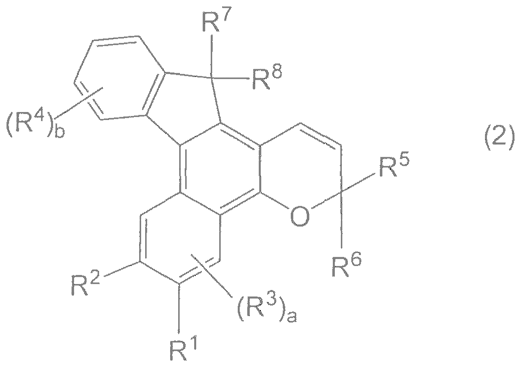 wherein R¹ and R² are as defined in the above formula (1), R³ is a hydroxyl group, alkyl group, haloalkyl group, cycloalkyl group, alkoxy group, amino group, heterocyclic group having a ring member nitrogen atom and bonded to a benzene ring bonded thereto via the nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group, R⁴ is a hydroxyl group, alkyl group, haloalkyl group, cycloalkyl group, alkoxy group, amino group, heterocyclic group having a ring member nitrogen atom and bonded to a benzene ring bonded thereto via the nitrogen atom, cyano group, nitro group, formyl group, hydroxycarbonyl group, alkylcarbonyl group, alkoxycarbonyl group, halogen atom, aralkyl group, aralkoxy group, aryl group, aryloxy group, thiol group, alkylthio group, alkoxyalkylthio group, haloalkylthio group, cycloalkylthio group, arylthio group, heteroarylthio group, hydroxysulfonyl group, alkylsulfonyl group, alkoxyalkylsulfonyl group, haloalkylsulfonyl group, cycloalkylsulfonyl group, arylsulfonyl group, heteroarylsulfonyl group, hydroxysulfinyl group, alkylsulfinyl group, alkoxyalkylsulfinyl group, haloalkylsulfinyl group, cycloalkylsulfinyl group, arylsulfinyl group and heteroarylsulfinyl group, R⁵ and R⁶ are each independently a group represented by the following formula (3): 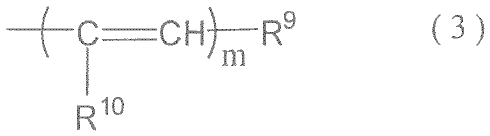 (R⁹ is an aryl group or a heteroaryl group, R¹⁰ is a hydrogen atom, alkyl group or halogen atom, and "m" is an integer of 1 to 3),
group represented by the following formula (4): 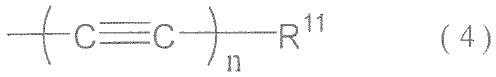 (R¹¹ is an aryl group or a heteroaryl group, and "n" is an integer of 1 to 3),
aryl group, heteroaryl group or alkyl group, R⁵ and R⁶ may form an aliphatic hydrocarbon ring together with carbon atoms bonded thereto, R⁷ and R⁸ are each independently a hydrogen atom, hydroxyl group, alkyl group, haloalkyl group, cycloalkyl group, alkoxy group, amino group, heterocyclic group having a ring member nitrogen atom and bonded to the 13-position carbon atom via the nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group, R⁷ and R⁸ may formed an aliphatic ring having 3 to 20 ring member carbon atoms, condensed polycyclic ring having an aromatic ring or aromatic hetero ring condensed to the aliphatic ring, hetero ring having 3 to 20 ring member atoms, or condensed polycyclic ring having an aromatic ring or an aromatic hetero ring condensed to the hetero ring together with the 13-position carbon atom bonded thereto, "a" is an integer of 0 to 2, "b" is an integer of 0 to 4, when "a" is 2, two R³' s may be the same or different, and when "b" is 2 to 4, a plurality of R⁴' s may be the same or different.

3. The chromene compound according to claim 1 to 2, wherein in the above formula (2), R⁷ and R⁸ form an aliphatic hydrocarbon ring together with the 13-positon carbon atom bonded thereto, and the aliphatic hydrocarbon ring has 4 to 20 ring member carbon atoms and may have at least one substituent selected from the group consisting of alkyl group, haloalkyl group, cycloalkyl group, alkoxy group, amino group, aralkyl group, aryl group and halogen atom.

4. The chromene compound according to any one of claims a 1 to 3, wherein the ratio (A_{Y}/A_{B}) of the color optical density (A_{Y}: value of λₘₐₓ of an absorption peak having a maximum absorption wavelength at 430 to 530 nm to the color optical density (A_{B}: value of λₘₐₓ) of an absorption peak having a maximum absorption wavelength at 550 to 650 nm is 0.80 to 2.00.

5. A photochromic curable composition comprising the chromene compound of any one of claims 1 to 4 and a polymerizable monomer.

6. A photochromic optical article having a polymer molded product comprising the chromene compound of any one of claims 1 to 4 dispersed therein as a constituent member.

7. An optical article having an optical substrate all or part of at least one surface of which is covered with a polymer film comprising the chromene compound of any one of claims 1 to 4 dispersed therein as a constituent member.

8. A naphthol compound represented by the following formula (5): 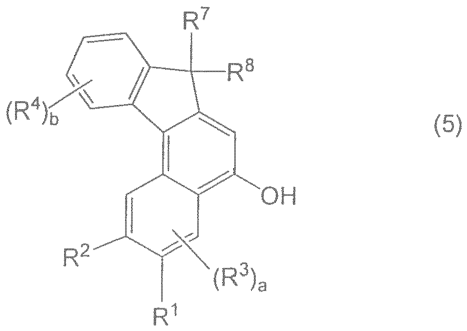 wherein R¹, R², R³, R⁴, R⁷, R⁸, "a" and "b" are as defined in the above formula (2).

## Patentansprüche

1. Chromenverbindung, die ein Gerüst hat, das durch die folgenden Formel (1) dargestellt ist: 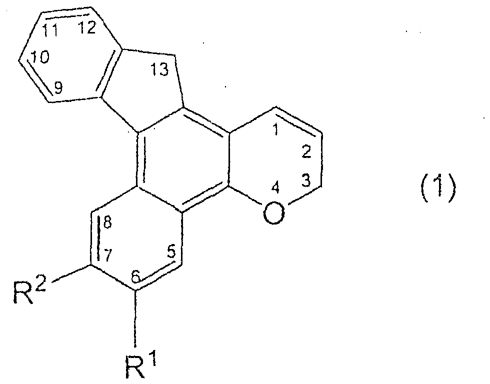 worin R¹ eine Arylgruppe oder eine Heteroarylgruppe ist und R² ein schwefelhaltiger Substituent ist, ausgewählt aus der Gruppe, bestehend aus einer Thiolgruppe, Alkylthiogruppe, Alkoxyalkylthiogruppe, Halogenalkylthiogruppe, Cycloalkylthiogruppe, Arylthiogruppe und Heteroarylthiogruppe.

2. Chromenverbindung gemäß Anspruch 1, die durch die folgende Formel (2) dargestellt ist: 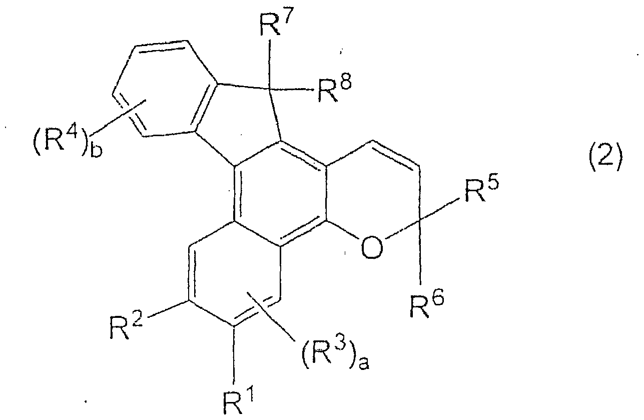 worin R¹ und R² wie in der obigen Formel (1) definiert sind, R³ eine Hydroxylgruppe, Alkylgruppe, Halogenalkylgruppe, Cycloalkylgruppe, Alkoxygruppe, Aminogruppe, heterocyclische Gruppe, die ein Ringgliedstickstoffatom hat und an einen Benzolring gebunden ist, der daran über das Stickstoffatom gebunden ist, Cyanogruppe, Nitrogruppe, Formylgruppe, Hydroxycarbonylgruppe, Alkylcarbonylgruppe, Alkoxycarbonylgruppe, ein Halogenatom, eine Aralkylgruppe, Aralkoxygruppe, Aryloxygruppe oder Arylgruppe ist, R⁴ eine Hydroxygruppe, Alkylgruppe, Halogenalkylgruppe, Cycloalkylgruppe, Alkoxygruppe, Aminogruppe, heterocyclische Gruppe, die ein Ringgliedstickstoffatom hat und an einen Benzolring gebunden ist, der daran über das Stickstoffatom gebunden ist, Cyanogruppe, Nitrogruppe, Formylgruppe, Hydroxycarbonylgruppe, Alkylcarbonylgruppe, Alkoxycarbonylgruppe, ein Halogenatom, eine Aralkylgruppe, Aralkoxygruppe, Arylgruppe, Aryloxygruppe, Thiolgruppe, Alkylthiogruppe, Alkoxyalkylthiogruppe, Halogenalkylthiogruppe, Cycloalkylthiogruppe, Arylthiogruppe, Heteroarylthiogruppe, Hydroxysulfonylgruppe, Alkylsulfonylgruppe, Alkoxyalkylsulfonylgruppe, Halogenalkylsulfonylgruppe, Cycloalkylsulfonylgruppe, Arylsulfonylgruppe, Heteroarylsulfonylgruppe, Hydroxysulfinylgruppe, Alkylsulfinylgruppe, Alkoxyalkylsulfinylgruppe, Halogenalkylsulfinylgruppe, Cycloalkylsulfinylgruppe, Arylsulfinylgruppe und Heteroarylsulfinylgruppe ist, R⁵ und R⁶ jeweils unabhängig eine Gruppe, dargestellt durch die folgende Formel (3): 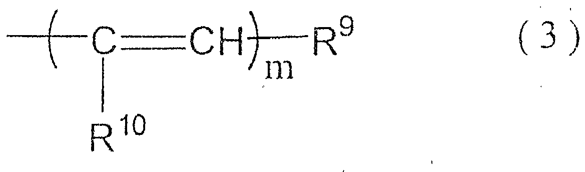 (R⁹ ist eine Arylgruppe oder eine Heteroarylgruppe, R¹⁰ ist ein Wasserstoffatom, eine Alkylgruppe oder ein Halogenatom und "m" ist eine ganze Zahl von 1 bis 3),
eine Gruppe, dargestellt durch die folgende Formel (4): 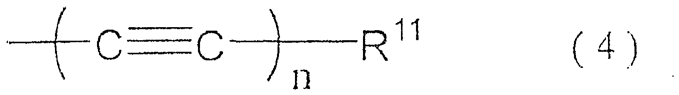 (R¹¹ ist eine Arylgruppe oder eine Heteroarylgruppe und "n" ist eine ganze Zahl von 1 bis 3),
eine Arylgruppe, Heteroarylgruppe oder Alkylgruppe sind, R⁵ und R⁶ zusammen mit den daran gebundenen Kohlenstoffatomen einen aliphatischen Kohlenwasserstoffring bilden können, R⁷ und R⁸ jeweils unabhängig ein Wasserstoffatom, eine Hydroxylgruppe, Alkylgruppe, Halogenalkylgruppe, Cycloalkylgruppe, Alkoxygruppe, Aminogruppe, heterocyclische Gruppe, die ein Ringgliedstickstoffatom hat und an das Kohlenstoffatom an der 13-Position über das Stickstoffatom gebunden ist, Cyanogruppe, Nitrogruppe, Formylgruppe, Hydroxycarbonylgruppe, Alkylcarbonylgruppe, Alkoxycarbonylgruppe, ein Halogenatom, eine Aralkylgruppe, Aralkoxygruppe, Aryloxygruppe oder Arylgruppe sind, R⁷ und R⁸ einen aliphatischen Ring, der 3 bis 20 Ringgliedkohlenstoffatome hat, einen kondensierten polycyclischen Ring, der einen aromatischen Ring oder aromatischen Heteroring, kondensiert an den aliphatischen Ring, hat, einen Heteroring, der 3 bis 20 Ringgliedatome hat oder einen kondensierten polycyclischen Ring, der einen aromatischen Ring oder einen aromatischen Heteroring, kondensiert an den Heteroring hat, zusammen mit dem daran gebundenen Kohlenstoffatom an der 13-Position bilden können, "a" eine ganze Zahl von 0 bis 2 ist, "b" eine ganze Zahl von 0 bis 4 ist, wenn "a" 2 ist, können zwei R³' s gleich oder verschieden sein und wenn "b" 2 bis 4 ist, können eine Vielzahl an R⁴'s gleich oder verschieden sein.

3. Chromenverbindung gemäß Anspruch 1 oder 2, wobei in der obigen Formel (2), R⁷ und R⁸ einen aliphatischen Kohlenwasserstoffring zusammen mit dem daran gebundenen Kohlenstoffatom an der 13-Position bilden und der aliphatische Kohlenwasserstoffring 4 bis 20 Ringgliedkohlenstoffatome hat und mindestens einen Substituenten haben kann, ausgewählt aus der Gruppe, bestehend aus einer Alkylgruppe, Halogenalkylgruppe, Cycloalkylgruppe, Alkoxygruppe, Aminogruppe, Aralkylgruppe, Arylgruppe und einem Halogenatom.

4. Chromenverbindung gemäß einem der Ansprüche 1 bis 3, wobei das Verhältnis (A_{γ}/A_{B}) der Farb-optischen Dichten (A_{γ}: Wert von λₘₐₓ) eines Absorptionspeaks, der eine maximale Absorptionswellenlänge bei 430 bis 530 nm hat, zu der Farb-optischen Dichte (A_{B}: Wert von λₘₐₓ) eines Absorptionspeaks, der eine maximale Absorptionswellenlänge bei 550 bis 650 nm hat, 0,80 bis 2,00 ist.

5. Photochrome härtbare Zusammensetzung, die die Chromenverbindung gemäß einem der Ansprüche 1 bis 4 und ein polymerisierbares Monomer umfasst.

6. Photochromer optischer Artikel, der ein geformtes Polymerprodukt, das die Chromenverbindung gemäß einem der Ansprüche 1 bis 4 darin dispergiert umfasst, als ein Bestandteils-Element hat.

7. Optischer Artikel, der ein optisches Substrat, bei dem die gesamte oder ein Teil mindestens einer Oberfläche mit einem Polymerfilm, der die Chromenverbindung gemäß einem der Ansprüche 1 bis 4 darin dispergiert umfasst, beschichtet ist, als ein Bestandteils-Element hat.

8. Naphtolverbindung, dargestellt durch die folgende Formel (5) : 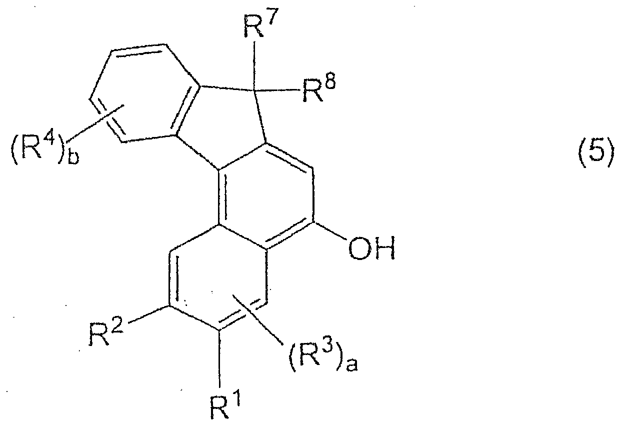 worin R¹, R², R³, R⁴, R⁷, R⁸, "a" und "b" wie in der obigen Formel (2) definiert sind.

## Revendications

1. Composé de chromène ayant un squelette représenté par la formule (1) suivante : 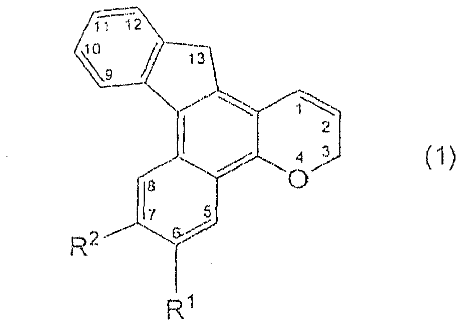 dans laquelle R¹ est un groupe aryle ou un groupe hétéroaryle, et R² est un substituant contenant du soufre sélectionné dans le groupe constitué d'un groupe thiol, d'un groupe alkylthio, d'un groupe alcoxyalkylthio, d'un groupe halogénoalkylthio, d'un groupe cycloalkylthio, d'un groupe arylthio et d'un groupe hétéroarylthio.

2. Composé de chromène selon la revendication 1, qui est représenté par la formule (2) suivante : 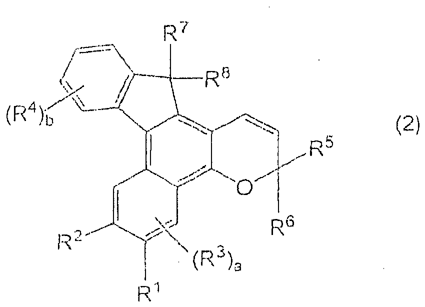 dans laquelle R¹ et R² sont tels que définis dans la formule (1) ci-dessus, R³ est un groupe hydroxyle, un groupe alkyle, un groupe halogénoalkyle, un groupe cycloalkyle, un groupe alcoxy, un groupe amino, un groupe hétérocyclique ayant un atome d'azote en tant qu'élément du cycle et lié à un cycle benzène lié à celui-ci par l'intermédiaire de l'atome d'azote, un groupe cyano, un groupe nitro, un groupe formyle, un groupe hydroxycarbonyle, un groupe alkylcarbonyle, un groupe alcoxycarbonyle, un atome d'halogène, un groupe aralkyle, un groupe aralcoxy, un groupe aryloxy ou un groupe aryle, R⁴ est un groupe hydroxyle, un groupe alkyle, un groupe halogénoalkyle, un groupe cycloalkyle, un groupe alcoxy, un groupe amino, un groupe hétérocyclique ayant un atome d'azote en tant qu'élément du cycle et lié à un cycle benzène lié à celui-ci par l'intermédiaire de l'atome d'azote, un groupe cyano, un groupe nitro, un groupe formyle, un groupe hydroxycarbonyle, un groupe alkylcarbonyle, un groupe alcoxycarbonyle, un atome d'halogène, un groupe aralkyle, un groupe aralcoxy, un groupe aryle, un groupe aryloxy, un groupe thiol, un groupe alkylthio, un groupe alcoxyalkylthio, un groupe halogénoalkylthio, un groupe cycloalkylthio, un groupe arylthio, un groupe hétéroarylthio, un groupe hydroxysulfonyle, un groupe alkylsulfonyle, un groupe alcoxyalkylsulfonyle, un groupe halogénoalkylsulfonyle, un groupe cycloalkylsulfonyle, un groupe arylsulfonyle, un groupe hétéroarylsulfonyle, un groupe hydroxysulfinyle, un groupe alkylsulfinyle, un groupe alcoxyalkylsulfinyle, un groupe halogénoalkylsulfinyle, un groupe cycloalkylsulfinyle, un groupe arylsulfinyle et un groupe hétéroarylsulfinyle, R⁵ et R⁶ sont chacun indépendamment un groupe représenté par la formule (3) suivante : 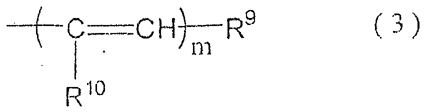 (R⁹ est un groupe aryle ou un groupe hétéroaryle, R¹⁰ est un atome d'hydrogène, un groupe alkyle ou un atome d' halogène, et « m » est un nombre entier de 1 à 3),
un groupe représenté par la formule (4) suivante : 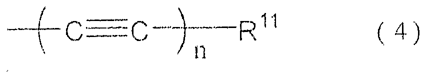 (R¹¹ est un groupe aryle ou un groupe hétéroaryle, et « n » est un nombre entier de 1 à 3,
un groupe aryle, un groupe hétéroaryle ou un groupe alkyle, R⁵ et R⁶ peuvent former un cycle hydrocarboné aliphatique conjointement aux atomes de carbone auxquels ils sont liés, R⁷ et R⁸ sont chacun indépendamment un atome d'hydrogène, un groupe hydroxyle, un groupe alkyle, un groupe halogénoalkyle, un groupe cycloalkyle, un groupe alcoxy, un groupe amino, un groupe hétérocyclique ayant un atome d'azote en tant qu'élément du cycle et lié à l'atome de carbone en position 13 par l'intermédiaire de l'atome d'azote, un groupe cyano, un groupe nitro, un groupe formyle, un groupe hydroxycarbonyle, un groupe alkylcarbonyle, un groupe alcoxycarbonyle, un atome d'halogène, un groupe aralkyle, un groupe aralcoxy, un groupe aryloxy ou un groupe aryle, R⁷ et R⁸ peuvent former un cycle aliphatique ayant de 3 à 20 atomes de carbone en tant qu'éléments du cycle, un cycle polycyclique condensé ayant un cycle aromatique ou un cycle hétéroaromatique condensé au cycle aliphatique, un hétérocycle ayant de 3 à 20 atomes en tant qu'éléments du cycle, ou un cycle polycyclique condensé ayant un cycle aromatique ou un cycle hétéroaromatique condensé à l'hétérocycle conjointement à l'atome de carbone en position 13 y étant lié, « a » est un nombre entier de 0 à 2, « b » est un nombre entier de 0 à 4, lorsque « a » vaut 2, deux R³ peuvent être identiques ou différents et, lorsque « b » vaut 2 à 4, une pluralité de radicaux R⁴ peuvent être identiques ou différents.

3. Composé de chromène selon la revendication 1 ou 2, dans lequel dans la formule (2) ci-dessus, R⁷ et R⁸ forment un cycle hydrocarboné aliphatique conjointement à l'atome de carbone en position 13 y étant lié, et le cycle hydrocarboné aliphatique a de 4 à 20 atomes de carbone en tant qu'éléments du cycle et peut avoir au moins un substituant sélectionné dans le groupe constitué d'un groupe alkyle, d'un groupe halogénoalkyle, d'un groupe cycloalkyle, d'un groupe alcoxy, d'un groupe amino, d'un groupe aralkyle, d'un groupe aryle et d'un atome d'halogène.

4. Composé de chromène selon l'une quelconque des revendications 1 à 3, dans lequel le rapport (A_{γ}/A_{B}) de la densité optique de couleur (A_{γ} : valeur de λₘₐₓ) d'un pic d'absorption ayant une longueur d'onde d'absorption maximale à 430 à 530 nm sur la densité optique de couleur (A_{B} : valeur de λₘₐₓ) d'un pic d'absorption ayant une longueur d'onde d'absorption maximale à 550 à 650 nm est de 0,80 à 2,00.

5. Composition photochrome durcissable comprenant le composé de chromène selon l'une quelconque des revendications 1 à 4 et un monomère polymérisable.

6. Article optique photochrome ayant un produit polymère moulé comprenant le composé de chromène selon l'une quelconque des revendications 1 à 4 dispersé à l'intérieur de celui-ci en tant qu'élément constitutif.

7. Article optique ayant un substrat optique dont la totalité ou une partie d'au moins une surface est revêtue d'un film polymère comprenant le composé de chromène selon l'une quelconque des revendications 1 à 4 dispersé à l'intérieur de celui-ci en tant qu'élément constitutif.

8. Composé de naphtol représenté par la formule (5) suivante : 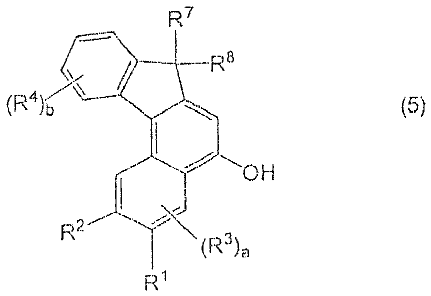 dans laquelle R¹, R², R³, R⁴, R⁷, R⁸, « a » et « b » sont tels que définis dans la formule (2) ci-dessus.
